(19) Europäisches Patentamt

European Patent Office

Office européen des brevets

(11) **EP 0 913 120 A1**

(12) **EUROPÄISCHE PATENTANMELDUNG**

(43) Veröffentlichungstag:
06.05.1999 Patentblatt 1999/18

(51) Int. Cl.$^6$: **A61B 5/00**

(21) Anmeldenummer: 97118971.7

(22) Anmeldetag: 30.10.1997

(84) Benannte Vertragsstaaten:
AT BE CH DE DK ES FI FR GB GR IE IT LI LU MC NL PT SE
Benannte Erstreckungsstaaten:
AL LT LV RO SI

(71) Anmelder:
- **Krauss, Manfred, Prof., Dr.-Ing.habil.**
  **D-09126 Chemnitz (DE)**
- **Grohmann, Gerald, Dr. med.**
  **07743 Jena (DE)**
- **Bilz, Dietrich, Dr. sc.**
  **12623 Berlin (DE)**

(72) Erfinder:
- **Krauss, Manfred, Prof., Dr.-Ing.habil.**
  **D-09126 Chemnitz (DE)**
- **Grohmann, Gerald, Dr. med.**
  **07743 Jena (DE)**
- **Bilz, Dietrich, Dr. sc.**
  **12623 Berlin (DE)**

(74) Vertreter:
**Kruspig, Volkmar, Dipl.-Ing. et al**
**Patentanwälte**
**Meissner, Bolte & Partner**
**Postfach 86 06 24**
**81633 München (DE)**

(54) **Vorrichtung und Verfahren zur nichtinvasiven Messung von Kreislauf-Parametern**

(57) Funktionszustände und Kennfunktionen des Kreislaufsystems und der Lunge werden im Zusammenwirken mit dem autonomen Nervensystem mit Hilfe der an sich bekannten Zwei-Wellenlängen-NIR-ROT-Remissions-Photoplethysmographie abgeleitet. Aus den erhaltenen Zeitfunktionen werden die Kennfunktionen peripherer Mikrozirkulations-Koeffizient, peripherer Volumen-Koeffizient, peripheres Vasodilatations-Maß, arterielle Sauerstoffsättigungs-Veränderung im Vergleich zu einer Bezugsmessung (z.B. von Ruhe- zu Hyperventilation) je Herzaktion als Zeitreihen-Tachogramm einschließlich dem der Herzperiodik ermittelt, ebenso die arteriolo-venoläre Sauerstoffdifferenz je Volumenpuls. Die nichtinvasiv erhaltenen Funktionen werden einer Auto- und/oder Kreuzkorrelationsbildung unterzogen, die daraus abgeleiteten Funktionszustände und Kennfunktionen gespeichert, mit Normalverläufen und -werten verglichen und/oder einer graphischen Darstellung unterzogen, so daß beispielsweise auch Aussagen über die Auswirkungen von Pharmaka oder Drogen gewonnen werden können und/oder Größen ableitbar sind, die zur Patientenüberwachung herangezogen werden können.

Fig. 1

S1...Sender1 (840nm)
S2...Sender2 (640nm)
E ... Empfänger
δ ... Temperatursensor

EP 0 913 120 A1

**Beschreibung**

Die Erfindung betrifft eine Vorrichtung und ein Arbeitsverfahren zum Betreiben der Vorrichtung zur nichtinvasiven Messung und Auswertung von Kreislauf-Kennfunktionen und Parametern.

Aus *Steinmann, J.* et al: "In-vivo-Messung der Sauerstoffsättigung des Blutes mit Quarzlichtleitern und einem optischen Vielkanalanalysator", Biomed.Technik 31 (1986), 246 - 251 ist es bekannt, mittels NIR-Rot-Remissions-Photoplethysmographie nichtinvasiv periphere Kreislaufparameter zu erfassen:

- Mit der Wellenlänge 840nm die relative Menge des Blutes bzw. der Erythrozyten im illuminierten Gewebe, unabhängig von dessen Oxygenierungsgrad, da bei Remissionsmessung in vivo der sog. $Hb/HbO_2$-isobestische Wellenlängenbereich bei 840 ... 850nm liegt. Als isobestisch wird dabei definitionsgemäß jener Wellenlängenbereich einer Strahlung bzw. des Lichts bezeichnet, bei dem unterschiedliche chemische Stoffe, z.B. Hämoglobin und Oxyhämoglobin, das durch diese Stoffe hindurchtretende Licht gleichermaßen schwächen, d.h. deren Extinktions- bzw. Schwächungskoeffizient gleich ist. Die NIR-Wellenlänge 840nm mißt somit relativ zum illuminierten Gesamtgewebe die Blut- bzw. Erythrozytenmenge, die die Mikrogefäße nach dem Fähraeus-Effekt in Form von Blutvolumenpulswellen passiert. Hierzu wird außerdem auf folgende Druckschriften verwiesen: US-PS 5,158,090; DD 293 199 A5, DD 298 677 A5; EP 90 104 729; DE 42 38 641 C2; DE 43 03 047.

- Mit der Wellenlänge 640nm andererseits die Oxygenierung der durch 840nm erfaßten relativen Blut bzw. Erythrozytenmenge. Theoretische Weiterentwicklungen und deren technische Umsetzungen erfolgten bisher kaum.

Ebenfalls ist bekannt [u.a. *Zander, R; Mertzlufft, F.O.*: Der Sauerstoff-Status des arteriellen Blutes. Basel, München, Pari, London, New York, New Delhi, Singapore, Tokyo, Sydney: Karger 1988, *Mertzlufft, F.*; *Zander, R.*: Spectrophotometric measurement of all hemoglobin derivatives in human blood. IUPS Proceedings XVI: 546 (1986)], daß mit der Pulsoxymetrie, die das Prinzip der Mehrwellenlängen-Transmissions-Photoplethysmographie realisiert [u. a. Ohmeda Biox 3740 Pulse Oximeter], die arterielle Sauerstoffsättigung $SaO_2$ des Blutes nichtinvasiv für ein bestimmtes Pulszahl-Intervall und damit bei einer entsprechenden Mittelungszeit (z.B. 3 bis 12 s bei Ohmeda Biox 3740 Pulse Oximeter) gemessen wird. Dabei wird diese oxymetrische Meßgröße definiert als prozentualer Anteil des oxygenierten Hämoglobins $HbO_2$ am Gesamthämaglobin ($HbO_2$ + Hb + sonstige Formen, wie Carbosyhämoglobin, Methämoglobin, Sulfhämoglobin):

$$SaO_2 = \frac{HbO_2}{HbO_2 \; + \; Hb \; + \; Sonstige}$$

[nach Handbuch Ohmeda Biox 3740 Pulse]

Andererseits wird allgemein die Sauerstoffsättigung als Verhältnis von $O_2$-Gehalt (in Vol.-%) zu $O_2$-Kapazität (in Vol.-%) definiert (*Netter, F.H.*: Farbatlanten der Medizin, Bd. 1: Herz, 3.überarb.u.erw. Auflage, The Ciba Collection of Medical Illustrations, 1993):

Ein Normalwert von 97% Sättigungsgrad des Blutes im Kapillarbett wird erhalten bei einem $O_2$-Gehalt von 20,4 Vol.-% und einer $O_2$-Kapazität von 21 Vol.-%.

Ebenso wird eine invasiv gemessene arterio-venöse Differenz $avDO_2$ der $O_2$-Gehalte eingeführt (*Schmidt, R.F.*; *Thews, G.*: Physiologie des Menschen. 23. Aufl., Springer-Verlage Berlin/Heidelberg/New York/London/Paris/Tokyo 1987), indem mit den durchschnittlichen Normalwerten für die arterielle $O_2$-Sättigung $SaO_2$ = 97% und die venöse $O_2$-Sättigung $SaO_2$ = 73% ein Gehalt an chemisch gebundenem Sauerstoff im arteriellen und venösen Blut von etwa 0,2 bzw. 0,15l $O_2$/l Blut invasiv gefunden wurde und damit sich etwa $avDO_2$ = 0,05l $O_2$/l Blut im Normalfall ergibt. Demnach werden normalerweise nur ca. 25% der gesamten $O_2$-Bindungskapazität des Blutes bei der Passage durch die Gewebekapillaren ausgeschöpft:

$$\text{SaO}_2 \text{ arteriell } = 0,97 = \frac{20,4 \text{ Vol.-\%}}{21 \quad \text{Vol.-\%}},$$

$$(21 \text{ Vol.-\% } : \text{ O}_2\text{-Kapazität})$$

$$\text{SaO}_2 \text{ venös } = 0,73 = \frac{15,3 \text{ Vol.-\%}}{21 \quad \text{Vol.-\%}},$$

d.h.

$$avDO_2 = 20,4 \text{ Vol.-\% } - 15,3 \text{ Vol.-\%}$$
$$\approx 5 \text{ Vol.-\%}$$
$$\triangleq 0,05l \text{ O}_2/l \text{ Blut}$$

Als relative arterio-venöse $O_2$-Differenz ergibt sich demnach

$$\text{SaO}_2 \text{ }_{arteriell} - \text{SaO}_2 \text{ }_{venös} = 0,24$$

bzw.

$$avDO_2 \text{ rel } = \frac{5 \text{ Vol.-\%}}{21 \text{ Vol.-\%}} = 0,24.$$

Auftretende Grenzen der Pulsoxymetrie-Transmissions-Messungen, Kalibrierungen und Meßgenauigkeiten werden ausführlich von *Zander, R. et al.* ("Der Sauerstoff-Status ..", s. vorn] behandelt.

Ausgehend vom Vorbekannten ist es Aufgabe der Erfindung, eine weiterentwickelte Vorrichtung und ein aussagefähigeres Arbeitsverfahren zum Betreiben der Vorrichtung zur nichtinvasiven Messung und Auswertung von zentralen und peripheren Kreislauf-Parametern anzugeben.

Die Lösung der Aufgabe erfolgt mit den Merkmalen der unabhängigen Patentansprüche, wobei die Unteransprüche mindestens zweckmäßige Ausgestaltungen und Weiterbildungen umfassen.

Die Erfindung geht von dem Grundgedanken aus, daß mit der NIR-ROT-Remissions-Photoplethysmographie die nichtinvasive Messung der Änderung der Oxygenierung des Blutvolumens in einem Mikrogefäßbereich sowie der sich einstellenden arteriolovenolären Differenz der Sauerstoffsättigung des Blutes je Herzaktion möglich ist, so daß von einer "Echtzeit-Oxymetrie" gesprochen werden kann.

Nach *Zander, R.; Mertzlufft, F.* "Der Sauerstoff-Status des arteriellen Blutes", Basel, Sydney: Karger 1988, S. 296, soll unter "Oxygenierung" verstanden werden die "reversible koordinative Bindung von Sauerstoff an das zweiwertige Eisen des Hämoglobins, wobei das desoxygenierte Hämoglobin in das oxygenierte Hämoglobin umgewandelt wird. Die Abspaltung von $O_2$ wird als Desoxygenation bezeichnet." Eine solche Abspaltung erfolgt nur im kapillären Bereich, so daß auch von einer "kapillären Desoxygenierung" gesprochen werden kann.

Eine mit der NIR-Wellenlänge 840 nm ermittelte Blutvolumenpulsation $x_1(t) = x_{NIR}(t)$ sowie die mit 640 nm erhaltene Zeitfunktion $x_2(t) = x_{ROT}(t)$ besitzen die gleiche Periodik, da sie von ein und derselben Herzaktion abgeleitet sind.

Es wird des weiteren als (ausreichende) Näherung vorausgesetzt, daß beide Wellenlängen aus etwa gleichen Gewebeeindringtiefen bzw. von annähernd gleichen Blutvolumina des illuminierten Gewebevolumens die Pulswellen $x_1(t)$, $x_2(t)$ ableiten. Ebenfalls wurde erkannt, daß bei Hyperämisierung des Meßortes beide Pulswellen nahezu deckungsgleich sind. Es liegt dann nach *Zander, R.* [s. vorn] eine große, unphysiologische Durchblutung mit einer arteriovenösen $O_2$-Differenz von annähernd Null vor, die Mikrogefäße sind weitgestellt. Bekannt ist auch, daß bei Patienten mit chronischer Anämie (z.B. Sichelzellanämie, Niereninsuffizienz) es zu einer gesteigerten Sauerstoffausnutzung kommt, d.h. zu einer höheren arteriovenösen Sauerstoff-Differenz des Oxyhämoglobins im Gewebe, zu einer Verschiebung der Sauerstoffbildungskurve nach rechts.

Gemäß der Erfindung werden die dargelegten Zusammenhänge in einer Kennfunktion erfaßt.

Im Sinne der Geometrie besteht zwischen den Signalen $x_1(t)$ und $x_2(t)$ eine "Ähnlichkeit", so daß quantitativ eine Korrelation bestimmbar ist.

Zur Ermittlung der (linearen) Korrelation zwischen diesen Signalen wird deren mittlere quadratische Abweichung $\overline{\varepsilon^2(t)}$ mit einem Kompensationsfaktor a wie folgt gebildet:

$$\overline{\varepsilon^2(t)} = \overline{[x_2(t) \cdot a - x_1(t)]^2} \to \text{Min},$$

d.h. bei optimalem Kompensationsfaktor $a_{opt}$ muß "maximale Ähnlichkeit" erreicht sein, die mittlere quadratische Differenz wird dann minimal, so daß daraus der Korrelationsfaktor bestimmt werden kann. Mathematisch-physikalischer Hintergrund dieser Gleichung ist, daß $x_1(t)$ das "Bezugssignal" bzw. den "Sollwert" (unabhängig vom oxymetrischen Zustand) darstellt, während $x_2(t)$ als "Istwert" (des oxymetrischen Zustandes) bezeichnet werden kann.

Man erhält

$$\overline{\varepsilon^2(t)} = a^2\,\overline{x_2^2(t)} + \overline{x_1^2(t)} - 2a\,\overline{x_1(t)x_2(t)}.$$

Durch Differentiation folgt

$$\frac{d\overline{\varepsilon^2(t)}}{da} = 0 = 2a_{opt} + \overline{x_2^2(t)} - 2\,\overline{x_1(t)x_2(t)},$$

bzw. ergibt sich für den optimalen Kompensationsfaktor

$$a_{opt} = \frac{\overline{x_1(t)\ x_2(t)}}{\overline{x_2^2(t)}}\ .$$

Durch Einsetzen und Umformen folgt für die minimale mittlere quadratische Differenz

$$\overline{\varepsilon^2(t)}_{min} = \overline{x_1^2(t)}\left[1 - \frac{\left(\overline{x_1(t)\cdot x_2(t)}\right)^2}{\overline{x_1^2(t)}\cdot\overline{x_2^2(t)}}\right]$$

bzw. in normierter Form

$$\frac{\overline{\varepsilon^2(t)}_{min}}{\overline{x_1^2(t)}} = 1 - \frac{\left[\overline{x_1(t)\cdot x_2(t)}\right]^2}{\overline{x_1^2(t)}\cdot\overline{x_2^2(t)}}\ .$$

Diese (normierte) minimale mittlere quadratische Differenz der Signale $x_1(t) = x_{NIR}(t)$, $x_2(t) = X_{ROT}(t)$ stellt offensichtlich das Quadrat eines Abstandsmaßes d dar, wie es auch die relative arterio-venöse Differenz $avdO_2$ [*nach Zander, R. et al.*, siehe vorn] ist, während als Korrelationsfaktor dieser Signale $x_1(t)$, $x_2(t)$ gesetzt wird

$$r = \frac{\overline{x_1(t) \cdot x_2(t)}}{\sqrt{\overline{x_1^2(t)} \cdot \overline{x_2^2(t)}}} = \frac{\overline{x_{NIR}(t) \cdot x_{ROT}(t)}}{\sqrt{\overline{x_{NIR}^2(t)} \cdot \overline{x_{ROT}^2(t)}}} \quad .$$

Zwischen dem Abstandsmaß d und dem Korrelationsfaktor r gilt demnach die Beziehung

$$d = \sqrt{1 - r^2} \quad ,$$

d.h. für r → 1 (Fall der Hyperämisierung) ist die Differenz d minimal (d → 0), während bei fehlender Korrelation r → 0 (theoretischer Grenzfall einer Anämie als Beispiel) die normierte Differenz maximal ist (d → 1).

Insbesondere nach Durchführung von funktionsdiagnostischen Modellversuchen bei reiner Stickstoff-Maskenatmung und nachfolgender Sauerstoff-Maskenatmung als zwei Extremzustände von Oxygenierung und Desoxygenierung des Blutvolumens in einem Mikrogefäßbereich (z.B. dem einer Zehenbeere) wurde überraschend erkannt:

-  Die zwischen der Blutvolumenpulsation $x_1(t)$ und dem oxymetrischen Signal $x_2(t)$ bestehende "Ähnlichkeit" (Korrelation) ermöglicht in der zu untersuchenden Funktionseinheit Mikrozirkulation eine quantitative Beurteilung des Zusammenwirkens von Blutfluß, Gefäßen sowie vasomotorischer Fasern peripherer Nerven. Deshalb soll hierfür von einem "peripheren Mikrozirkulations-Koeffizienten pMC" gesprochen werden, d.h.

$$r \triangleq pMC.$$

-  Der pMC-Grad (Korrelationsfaktor, Ähnlichkeitsmaß) kann je Herzaktion n bestimmt werden (also für jeden Blutvolumenpuls n), so daß formuliert werden kann

$$r(n) = pMC(n) = \frac{\overline{x_{NIR}(n) \cdot x_{ROT}(n)}}{\sqrt{\overline{x_{NIR}^2(n)} \cdot \overline{x_{ROT}^2(n)}}} \quad .$$

Als mittlerer pMC-Grad (Korrelationsfaktor r) für eine bestimmte Meßdauer (Vorliegen von N Volumenpulsen bzw. N Herzaktionen) gilt

$$\overline{r(n)} = \frac{1}{N} \sum_{n=1}^{N} r(n) = \overline{pMC(n)} = \frac{1}{N} \sum_{n=1}^{N} pMC(n) \quad .$$

-  Das eingeführte Abstandsmaß d korreliert mit der relativen arteriolo-venolären $O_2$-Differenz $avdO_2^*$ des Blutes und läßt sich je Herzaktion n nichtinvasiv und quantitativ bestimmen:

$$avdO_2^*(n) \triangleq d(n) = \sqrt{1 - [pMC(n)]^2} \quad .$$

Als Normalbereiche wurden ermittelt:

$$pMC = 97 \ldots 99\%,$$

$$d = 14 \ldots 24\%.$$

Es wurde ebenfalls erkannt, daß bei vertiefter Atmung (Hyperventilation) als Funktionstest mit ausreichender Genauigkeit

$$avdO_2{}^* = d \approx avdO_2$$

gesetzt werden kann.

- Ist im Gegensatz zur NIR-Blutvolumenpulsation im oxymetrischen Signal keine Periodik ersichtlich, dann kann z.B. eine Veränderung der Erythrozyten vorliegen (z.B. bei massiv erhöhtem mittleren zellulären Erythrozytenvolumen, Spätstadium der Sichelzellanämie). Der ermittelte pMC-Grad ist anormal klein, so daß anormal hohe arteriolovenoläre $O_2$-Differenzen des Blutes auftreten.

Wird das bekannte 2-Wellenlängen-Remissions-Verfahren auf die Verhältnisse bei der Lunge angewandt, indem der Sensor "lungennah" am Oberkörper befestigt wird, so läßt sich je Atmungsperiode n* der pMC-Grad für die Lunge ermitteln:

$$r(n^*)_{Lunge} = \frac{\overline{x_{NIR}(n^*) \cdot x_{ROT}(n^*)}}{\sqrt{\overline{x^2_{NIR}(n^*) \cdot x^2_{ROT}(n^*)}}} = pMC(n^*)_{Lunge}$$

bzw. als arithmetischer Mittelwert bei N* Atmungsperioden

$$\overline{r(n^*)} = \frac{1}{N^*} \sum_{n^*=1}^{N^*} r(n^*) = \overline{pMC(n^*)} \quad .$$

Aus den r(n) = pMC(n) sowie r(n*) = pMC(n*) sollen erfindungsgemäß die absoluten und relativen Standardabweichungen sowie die in den Verläufen auftretenden mittleren Perioden (abgeleitet aus den Autokorrelationsfunktionen) als weitere Gütemaße im Kreislauf-System bestimmt werden.

Bezieht man die Standardabweichung (Streuung)

$$S_{\Delta pMC} = \sqrt{\frac{1}{N-1} \sum_{n=1}^{N} [pMC(n) - \overline{pMC(n)}]^2}$$

auf den Mittelwert $\overline{pMC(n)}$, so erhält man eine "pCM-Variabilität" pMCV:

$$pCMV = \frac{S_{\Delta pMC}}{\overline{pMC}} \quad .$$

Eine solche Kenngröße wird analog für die Lunge ermittelt.

Es ist bereits gezeigt worden, daß ohne zu kalibrieren durch Ermittlung der Volumenpulsfläche der mikrovaskulären Blutvolumenpulsation ein "relatives (mikrovaskuläres) Füllungsvolumen" FV und damit seine Änderung je Herzaktion n an einer Meßstelle (einem Mikrogefäßbereich) bestimmt werden kann. Dabei wird die Fläche eines jeden Volumenpulses auf den mittleren Flächeninhalt aller Pulsationen während einer Meßzeit (z.B. 64 s) bezogen und als Tachogramm dargestellt (relatives Füllungsvolumen 1. Art).

Diese Darstellung wird sowohl für das 840nm-Blutvolumen als auch für das 640nm-oxymetrische Signal vorgenommen. Dabei sind bei normaler Oxygenierung beide Verläufe nahezu deckungsgleich

Aus den erhaltenen Verläufen $FV1_{840nm}$ (NZR-Signal, mikrovaskuläres Blutvolumen) sowie $FV1_{640nm}$ (Rot-Signal, oxymetrisches Signal) läßt sich, wie ebenfalls erkannt wurde, ein "Ähnlichkeitsgrad" bzw. Korrelationsfaktor ableiten, der sich als normierte Kovarianz $r_{FV1\ NIR,\ Rot}$ abbildet und "peripherer Volumen-Koeffizient" $pVC_1$, genannt werden soll:

$$pVC_1 = r_{FV1\ NIR,\ Rot} \cdot$$

Aus den Verläufen der Füllungsvolumina 1. Art soll erfindungsgemäß des weiteren der "periphere Volumen-Koeffizient" $pVC_2$ als Mittelwert abgeleitet werden

$$\overline{pVC_2} = \frac{1}{N} \sum_{n=1}^{N} FV1_{NIR}(n) \cdot FV1_{ROT}(n)$$

sowie die zugehörige Standardabweichung.

Ebenso wurde als periphere Kennfunktion $pVC_3$ der mittlere Zusammenhang

$$\overline{pVC_3} = \frac{1}{N} \sum_{n=1}^{N} \frac{FV1_{NIR}(n)}{FV1_{ROT}(n)}$$

einschließlich Standardabweichung dieses Quotientenverhältnisses erkannt.

Sollen die Füllungsvolumina unterschiedlicher Meßfiles, also zu unterschiedlichen Zeitpunkten, verglichen werden, so läßt sich durch Division der Volumenpulsflächen der aktuellen Messung und dem Pulsflächen-Mittelwert der Bezugsmessung (Bezugsmeßfiles genannt) die relative Veränderung sowohl vom Blutvolumen als auch vom oxymetrischen Signal darstellen (relativen Füllungsvolumen 2 Art) , ohne hierzu Merkmalszuordnungen zu treffen. Bestimmt man nun analog zum 840nm-Blutfüllungsvolumen das oxymetrische 640nm-Füllungsvolumen 2.Art, so läßt sich, wie erfindungsgemäß erkannt wurde, durch Vergleich beider Mittelwerte (Mittelwert vom 640nm-Füllungsvolumen größer, gleich oder kleiner als der Mittelwert vom 840nm-Blutvolumen) die Veränderung der arteriellen Sauerstoffsättigung am Meßort im Vergleich zur Bezugsmessung darstellen (z.B. von Ruhe- zu Hyperventilation oder durch entsprechende Therapieverfahren).

Aus Gründen der besseren Anschaulichkeit sind dabei sowohl die mikrovaskuläre Blutvolumenpulsation als auch das zugehörige oxymetrische Signal gleichphasig dargestellt, was an sich nicht den biologischen Gegebenheiten entspricht. Dadurch muß in Kauf genommen werden, daß ein Größerwerden des 640nm-Füllungsvolumens bezüglich des 840nm-Blutfüllungsvolumens einer Reduzierung der Oxygenierung des Blutvolumens an der Meßstelle entspricht, ein Kleinerwerden im Vergleich zum 840nm-Verlauf eine Zunahme der Oxygenierung bedeutet (Normalfall bei Hyperventilation).

Als quantitatives Maß für eine Reduzierung der arteriellen $O_2$-Sättigung am Meßort (z.B. bei Stickstoff-Maskenatmung) während der aktuellen Messung gegenüber einer Bezugsmessung soll das Verhältnis

$$V = \frac{840nm\text{-}Füllungsvolumen\ 2.Art}{640nm\text{-}Füllungsvolumen\ 2.Art}$$

definiert werden. Es läßt sich je Herzaktion n ermitteln.

Bei reduzierter arterieller $O_2$-Sättigung an einem peripheren Meßort gilt folglich für die Differenz $\Delta FV2$ der Füllungsvolumina

$$\Delta FV2 = FV2_{ROT} - FV2_{NIR} > 0$$
$$= FV2_{640nm} - FV2_{840nm} > 0,$$

d.h. $\Delta FV2$ ist ein Maß für die $O_2$-Reduzierung. Je größer $\Delta FV2$, desto größer ist die Reduzierung der arteriellen $O_2$-Sättigung am Meßort und umgekehrt.

Durch Normierung erhält man

$$\frac{\Delta FV2}{FV2_{ROT}} = \frac{\Delta FV2}{FV2_{640nm}} = 1 - \frac{FV2_{NIR}}{FV2_{ROT}}$$

$$= 1 - V.$$

Damit läßt sich die prozentuale arterielle $O_2$-Sättigungs-Reduzierung im Vergleich zu einer Bezugsmessung ermitteln:

$$\Delta O_{2Reduzierung} = \Delta SaO_{2Reduzierung}$$

$$= \frac{\Delta FV2}{FV2_{640nm}} \cdot 100 \ [\%] = 100 \ [1-V] \ \%.$$

Da das Verhältnis V je Herzaktion ermittelbar ist, ist $\Delta O_2$ ebenfalls je Herzaktion n bestimmbar. Gleichfalls sind Mittelwert, Streuung (Standardabweichung) sowie durch Bildung der Autokorrelationsfunktion $\Phi\Delta O_2(m)$ die auftretenden mittleren Perioden m ableitbar.

Tritt im Vergleich zu einer Bezugsmessung eine Erhöhung der arteriellen $O_2$-Sättigung an einem peripheren Meßort auf (z.B. bei Hyperventilation), dann folgt für die Differenz $\Delta FV2$ der Füllungsvolumina, wie erkannt wurde,

$$\Delta FV2 = FV2_{ROT} - FV2_{NIR} < 0$$

bzw.

$$\Delta FV2^* = FV2_{NIR} - FV2_{ROT} > 0,$$

d.h. je größer die Differenz $\Delta FV2^*$, desto größer ist die Erhöhung der arteriellen $O_2$-Sättigung am Meßort. Bei Normierung erhält man

$$\frac{\Delta FV2^*}{FV2_{ROT}} = \frac{\Delta FV2^*}{FV2_{640nm}} = \frac{FV2_{NIR}}{FV2_{ROT}} - 1$$

$$= V - 1.$$

Für die prozentuale Erhöhung der arteriellen $O_2$-Sättigung folgt demnach

$$\Delta O_{2Erhöhung} = \Delta SaO_{2Erhöhung}$$

$$= \frac{\Delta FV2^*}{FV2_{640nm}} \cdot 100 \ [\%] = 100 \ [V-1] \ \%,$$

wobei ebenso eine Ermittlung je Herzaktion möglich ist.

Medizinisch ist bekannt, daß mit zunehmendem Lebensalter sich der Dehnungsgrad der Gefäße reduziert. Es wurde erkannt, daß bei einer auftretenden Vasodilatation (durch entsprechende Pharmaka), d.h. $FV2_{NIR} > 1$, sich als Differenz $\Delta FV2 = FV2_{ROT} - FV2_{NIR} \geq 0$ ergibt, d.h. die Vasodilation der Gefäße ist mit einer Reduzierung der arteriellen $O_2$-Sättigung am Meßort verbunden. Als "Güte der Vasodilatation" wird das Verhältnis

$$\frac{\Delta FV2}{FV2_{NIR}} = \frac{FV2_{ROT} - FV2_{NIR}}{FV2_{NIR}}$$

$$= \frac{1 - V}{V} \rightarrow Minimum$$

erkannt, d.h. durch Minimierung des Pharmakaeinsatzes soll eine maximale Gefäßdehnung erreicht werden. Diese Beziehung wird "peripheres Vasodilatations-Maß" pVM genannt.

Die durch eine Therapie (als Beispiel) hervorgerufene Veränderung des peripheren Strömungwiderstandes $\Delta PW$ läßt sich (bei Konstanz des Druckabfalls im Kreislaufsystem) im Sinne von *Burton, A.C.* [*Burton, A.C.*: Physiologie und Biophysik des Kreislaufs. F.K. Schattauer, Stuttgart/New York 1969] durch Ermttlung des Verhältnisses Pulsperiodik / $FV2_{NIR}$ je Volumenpuls n der aktuellen Messung zu dem einer Bezugsmessung bestimmen. Es gilt für die NIRP-Methode, wie erfinderisch erkannt wurde, die Beziehung

$$\Delta PW(n) \, [\%] = \frac{\dfrac{T_H(n)}{FV2_{NIR}(n)} \Big/ aktuelle \; Messung}{\dfrac{T_H(n)}{FV2_{NIR}(n)} \Big/ Bezugs\text{-} \; messung} \cdot 100$$

wobei $\overline{T_H(n) / FV2_{NIR}(n)}$ der Mittelwert der Bezugsmessung ist.

Es wurde erkannt, daß die Koeffizienten und Maße nicht nur in der Peripherie des großen Kreislaufsystems ermittelbar sind, sondern analog auf den kleinen Kreislauf (d.h. den Beginn der $O_2$-Übertragungsstrecke, die Lunge) übertragen werden können, wie bei dem pMC-Grad gezeigt wurde.

Erfindungsgemäß sollen pMC, pVC, pVM, $\Delta O_2$ des weiteren auf die Verhältnisse der dynamischen Systemdiagnostik angewendet werden.

Mit Hilfe der bekannten Leistungsspektralanalyse der Herzfrequenzvariabilität [u.a. *Esperer, H.-D.*: Die Herzfrequenzvariabilität, ein neuer Paramter für die nichtinvasive Risikostratifizierung nach Myokardinfarkt und arrhythmogener Synkope. Herzschr. Elektrophys. 3: 1-6 (1992)] ist es möglich, Störungen der parasympathischen und sympathischen Innervation des Herzens zu erfassen. Dabei wird aus dem Tachogramm der Herzperiodendauern $T_H = f(n)$, abgeleitet aus den RR-Abständen im EKG, bei einer Meßzeit von z.B. 500s das "Leistungspektrum der Herzfrequenzvariabilität" mit charakteristischen Frequenzbereichen ermittelt und bewertet. Methodisch-technische Schwierigkeiten (Signal-Averaging, Fourier-Analyse) setzen dabei gewisse Grenzen.

Aus den NIR-Blutvolumenpulsationen (Meßort Fingerbeere) läßt sich - analog zum RR-Abstand im EKG - mit ausreichender Genauigkeit (Amplitudenauflösung des zu erfassendeen NIR-Signals 16 bit, mindestens 128 Abtastungen/s als Zeitauflösung) das "Tachogramm der Pulsperiodendauern (Herzperiodendauern)" als eine kardiovaskuläre Kennfunktion ableiten, indem für jede Herzaktion n die zugehörige Dauer $T_H$ graphisch aufgetragen wird. In einem solchen Verlauf mit etwa 60 - 100 Herzaktionen für ein Meßintervall sind schon mit bloßem Auge bestimmte Regelmäßigkeiten, also Perioden bzw. Abhängigkeiten zwischen den Herzaktionen (z.B. Auftreten der respiratorischen Sinusarrhythmie im Normalfall) ersichtlich, aber auch neben der mittleren Herzperiodendauer (Herzfrequenz) die auftretenden Abweichungen von diesem Wert (Herzfrequenzvariabilität in der Kardiologie genannt). Aus diesem Tachogramm läßt sich die Autokorrelationsfunktion als eine Mittelwertfunktion ableiten, aus der unmittelbar und quantitativ ersichtlich sind:

- auftretende mittlere Perioden zwischen den Herzaktionen,
- Herzfrequenzvariabilität.

Eine solche Mittelung ist ebenso bei den eingeführten peripheren Kennfunktionen Füllungsvolumina FV(n), pMC(n), pVM(n) und $\Delta O_2(n)$ möglich, wie erfinderisch erkannt wurde und nachfolgend für FV(n) und pMC(n) gezeigt wird.

Als Abweichung des relativen Füllungsvolumens FV(n) vom Mittelwert $\overline{FV(n)} = 1$ gilt analog zu $T_H = f(n)$

$$\Delta FV(n) = FV(n) - \overline{FV(n)}$$
$$= FV(n) - 1.$$

Damit folgt für die zu ermittelnde Autokorrelationsfunktion der Algorithmus analog zu $\Phi_{\Delta TH}(m)$

$$\Phi_{\Delta FV}(m) = \overline{FV(n)\ FV(n\text{-}m)} - 1.$$

Bei m=0 erhält man die "Füllungsvolumenvariabilität" FV V

$$FV\ V = \sqrt{\ \Phi_{\Delta FV}(m=0)}\ ,$$

während im Verlauf von $\Phi_{\Delta FV}(m)$ die mittleren Füllungsperioden (z.B. Atmungsdominanz im Normalfall, langwellige Grundperioden bei erhöhtem Sympathikotonus) ersichtlich sind. Analog gilt für die Abweichung des peripheren Mikrozirkulations-Koeffizienten pMC(n) vom arithmetischen Mittelwert $\overline{pMC(n)}$

$$\Delta pMC(n) = pMC(n) - \overline{pMC(n)}$$

sowie für die Autokorrelationsfunktion

$$\Phi_{\Delta pMC}(m) = \overline{pMC(n)\ pMC(n\text{-}m)} - \left[\overline{pMC(n)}\right]^2 .$$

Ebenfalls folgen aus $\Phi_{\Delta pMC}(m=0)$ die "pMC-Variabilität" pMC V

$$pMCV\ \ =\ \frac{\sqrt{\overline{\Phi_{\Delta pMC}(m=0)}}}{\overline{pMC(n)}}\ ,$$

sowie aus dem Verlauf $\Phi_{\Delta pMC}(m)$ die auftretenden mittleren Perioden.

Bekanntlich wird beim Elektrokardiogramm (EKG) die Herzstromkurve aufgezeichnet, die bei jeder Herzaktion gewonnen werden kann. Um eine genauere EKG-Beurteilung zu ermöglichen, werden dabei minimal 12 sogenannte Standard-Ableitungen (6 Extremitäten- und 6 Thorax-Ableitungen) aufgezeichnet und bewertet. So werden mit den 6 Extremitäten-Ableitungen die elektromotorischen Phänomene des Herzens auf der frontalen Ebene dargestellt (Betrachtung des Herzens von "vorn nach hinten"), während mit den Thorax-Ableitungen die elektromotorischen Phänomene von "oben nach unten" betrachtet werden. Demnach ermöglicht die Kombination von Extremitäten- und Thorax-Ableitungen, die elektromotorischen Kräfte des Herzens auch räumlich darzustellen.

Davon ausgehend, wie erfinderisch erkannt wurde, läßt sich durch jeweilige Kombination von 2 Ableitungen innerhalb der Extremitäten- und Thorax-Ableitungen, jedoch auch zwischen diesen Ableitungen, die dabei auftretende "Ähnlichkeit" als Korrelationsfaktor je Herzaktion analog zu pMC darstellen, Mittelwerte und Variabilität ermitteln und mit Normalverläufen und -werten vergleichen. Die Darstellung der Mittelwerte kann auch als farbiges Mapping erfolgen, um Normal- von Nichtnormalzuständen optisch eindeutiger abgrenzen zu können.

In einer weiteren Ausführungsform der Erfindung werden die Zustandsfunktionen $T_H$=f(n), FV=f(n), pMC=f(n), pVC=f(n), pVM=f(n), $\Delta O_2$=f(n), $T_H(n) \cdot FV(n)$=f(n) sowie $T_H$/FV=f(n) untereinander kreuzkorreliert, d.h. Kreuzkorrelationsfaktoren sowie mittlere Perioden nach dem Bildungsalgorithmus

$$\Phi_{xy}(m) = \overline{x(n)\ y(n\text{-}m)}$$

bestimmt. Damit erfolgt ein Vergleich mit Normalverläufen und -kennwerten.

Die Erfindung soll anhand von Ausführungsbeispielen und von Figuren näher erläutert werden.

Hierbei zeigen

Fig. 1      das Meßprinzip der NIR-ROT-Remissions-Photoplethysmographie (NIRP-Methode),

Fig. 2      Volumenpulsationen im Mikrogefäßbereich "Fingerbeere" bei einem 28jährigen liegenden Normalprobanden mit Ruheventilation, abgeleitet nach der NIRP-Methode,

a) mikrovaskuläre Blutvolumenpulsation [ermittelt mit der Wellenlänge 840nm], bestehend aus den Teilkomponenten $x_I(t)$, $x_{II,III,...N}(t)$ und bei einer Meßzeit von 64s,

b) in einem 4s-Zeitfenster mit charakteristischen Zeitparametern [$T_H$ = Puls/Herzperiodendauer, $T_G$ = Gipfelzeit, $T_{AG}$ = arterielle Grundschwingung, $T_D$ = Dikrotiezeit, $T_A$ = Abfallzeit] sowie der Pulsfläche $F_{20}$ als Beispiel für eine Blutvolumenmenge dieses Pulses.

c) Darstellung der mikrovaskulären Blutvolumenpulsation (Kanal 1) sowie des zugehörigen oxymetrischen Signals (Kanal 2) in einem 4s-Zeitfenster,

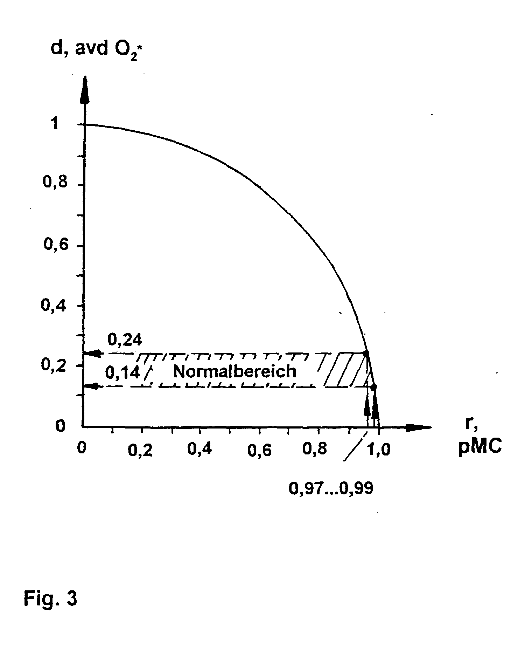

Fig. 3   ein aus einem Modell abgeleiteter Zusammenhang zwischen dem Signal-Abstandsmaß d [entspricht der relativen arteriolo-venolären Differenz $avdO_2^*$ der $O_2$-Sättigung des Blutes] und dem Korrelationsfaktor r (peripherer Mikrozirkulationskoeffizient pMC) der mikrovaskulären Blutvolumenpulsation sowie dem oxymetrischen Signal einschließlich ermittelte Normalbereiche,

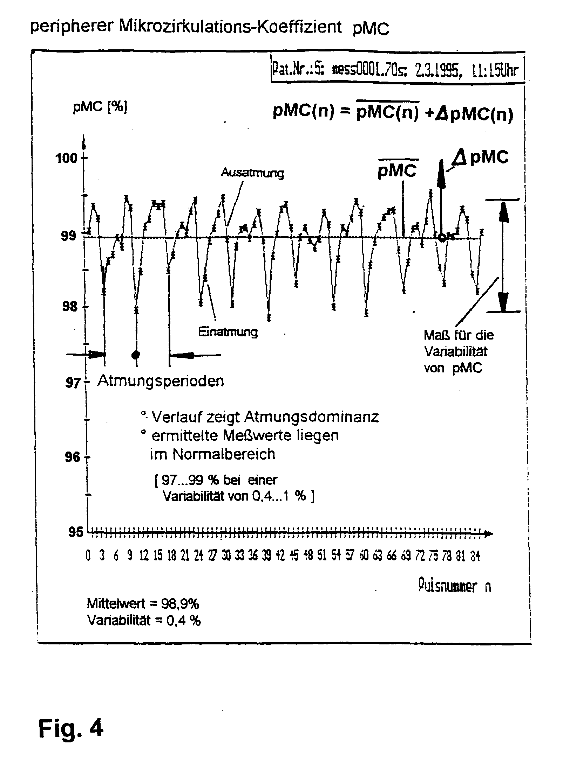

Fig. 4   den Verlauf von pMC bei einem 45jährigen Normalprobanden am Meßort Fingerbeere (liegende Position, Ruheventilation),

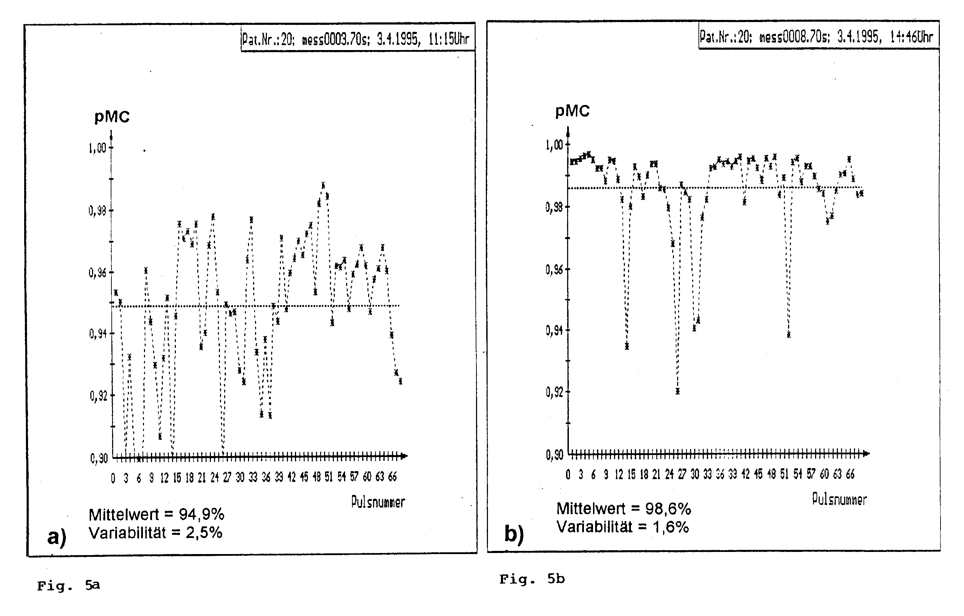

Fig. 5   den Verlauf von pMC a) vor und b) nach einer Infusion mit Prostavasin bei einem liegenden Patienten mit pAVK (Meßort Großzehe) und Ruheatmung,

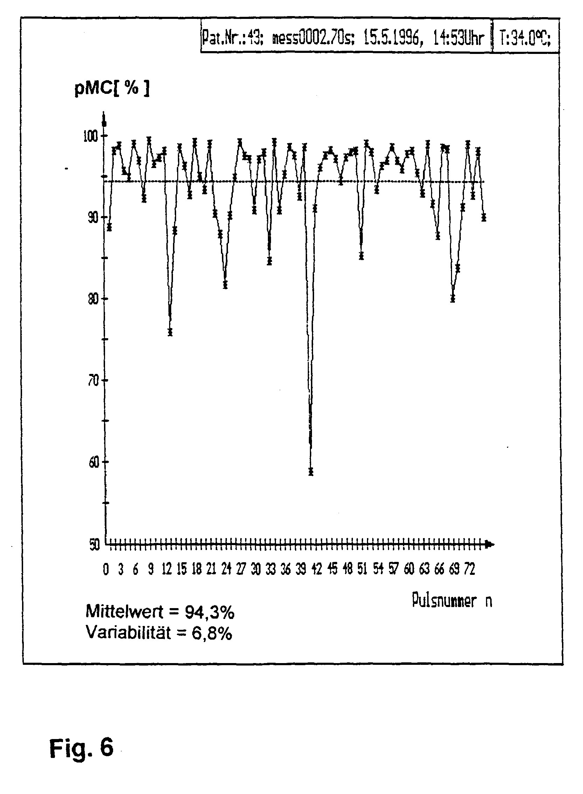

Fig. 6   den Verlauf von pMC bei einem 79jährigen liegenden Patienten mit Linksherzinsuffizienz und Ruheatmung (Meßort Fingerbeere),

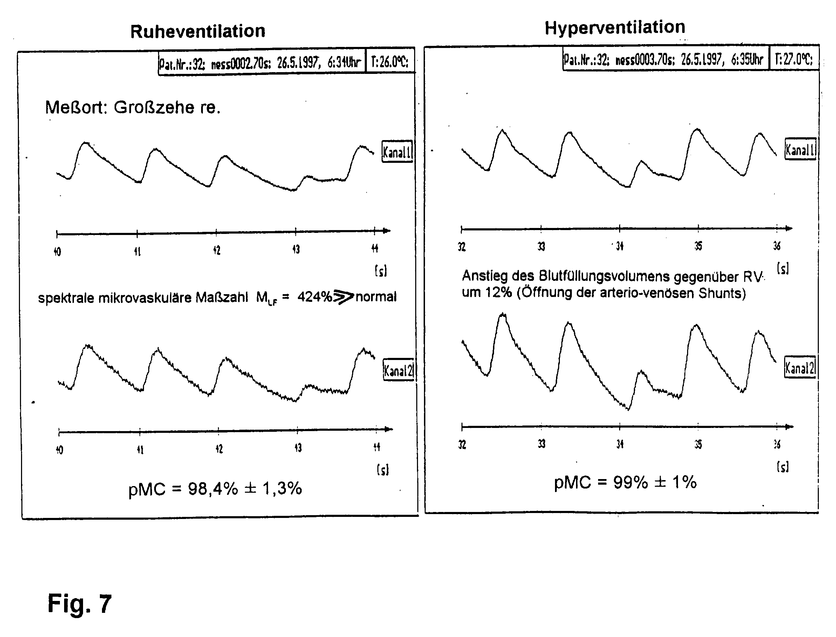

Fig. 7   das Auftreten einer diabetischen Polyneuropathie bei einem 54jährigen Typ I-Diabetiker, abgeleitet aus dem Funktionstest Ruhe- und Hyperventilation (Meßort Großzehe re.),

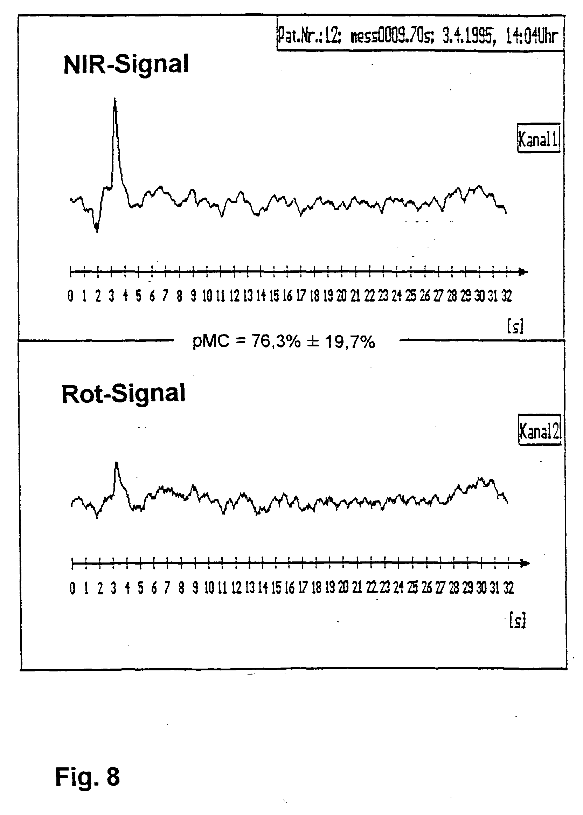

Fig. 8   eine nichtnormale Blutvolumenpulsation am Meßort Großzehe bei einem 85jährigen Patienten mit pAVK sowie ermittelter pMC-Grad,

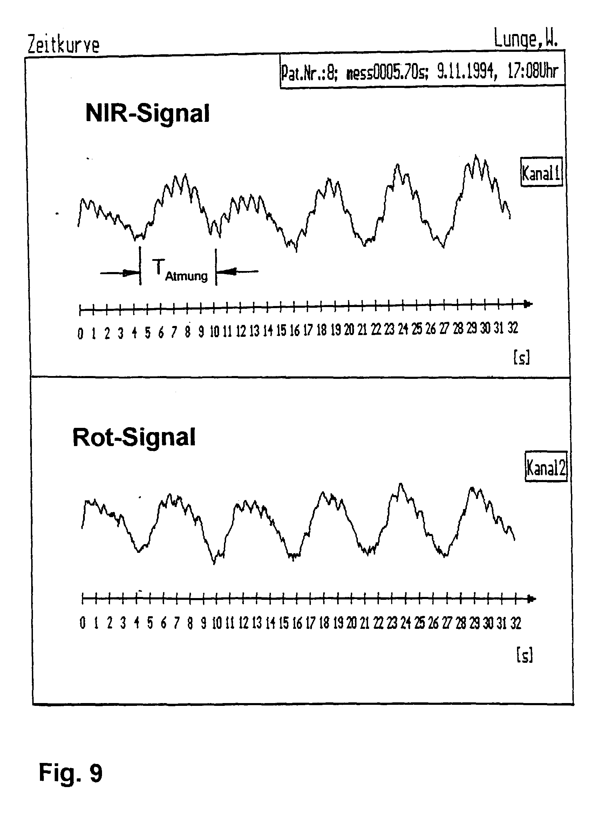

Fig. 9   Remissions-Photoplethysmographie-Signale am Meßort Oberkörper/Lungennähe,

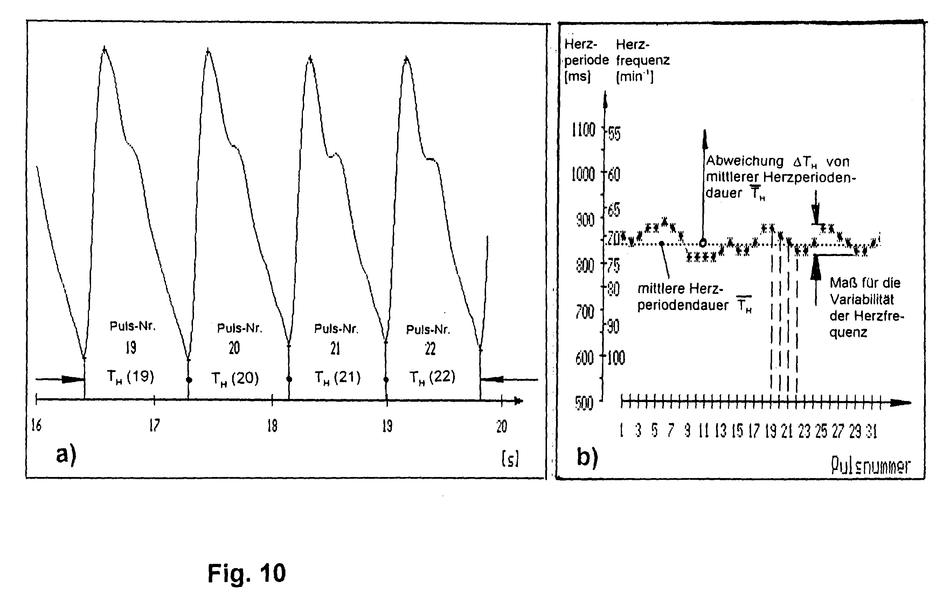

Fig. 10   die Ableitung des Tachogramms der Pulsperiodendauern (Herzperiodendauern) aus der mikrovaskulären Blutvolumenpulsation,

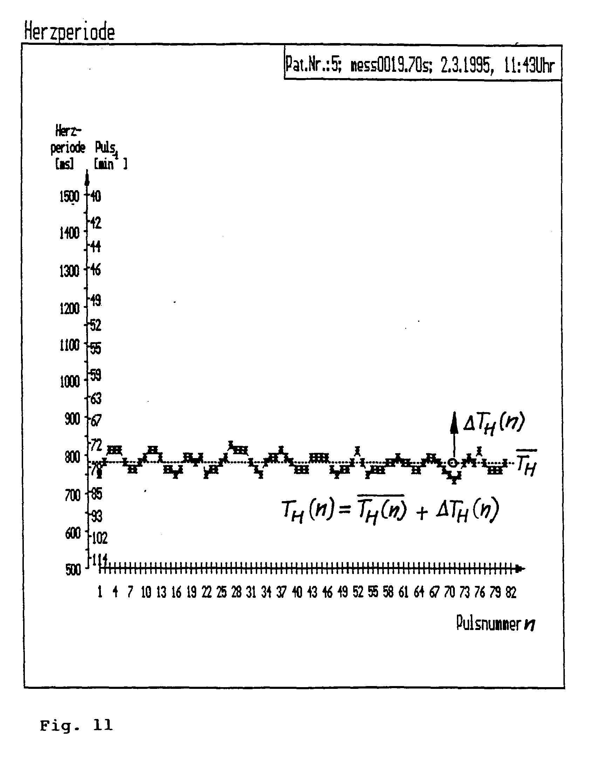

Fig. 11   das Tachogramm $T_H = f(n)$ eines 40jährigen Normalprobanden,

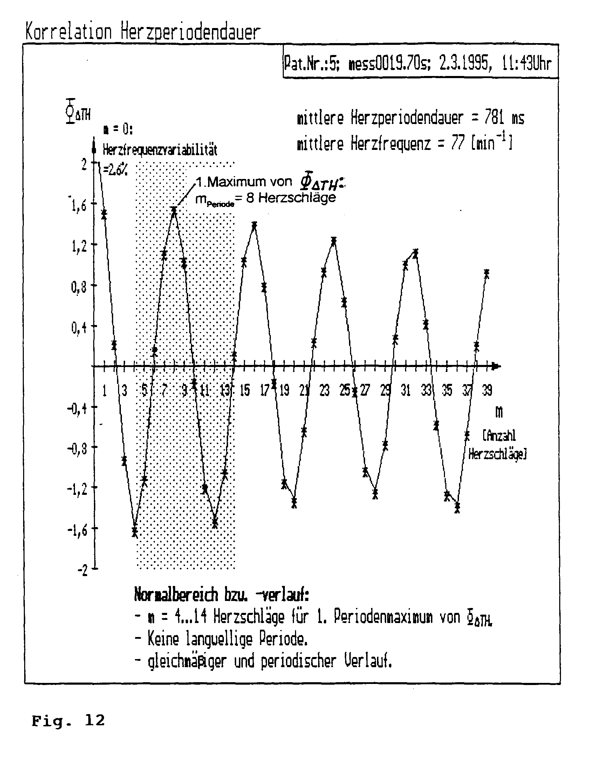

Fig. 12   die zu Fig. 11 gehörende Autokorrelationsfunktion $\Phi_{ATH}(m)$ einschließlich Kenn- und Normalwerten,

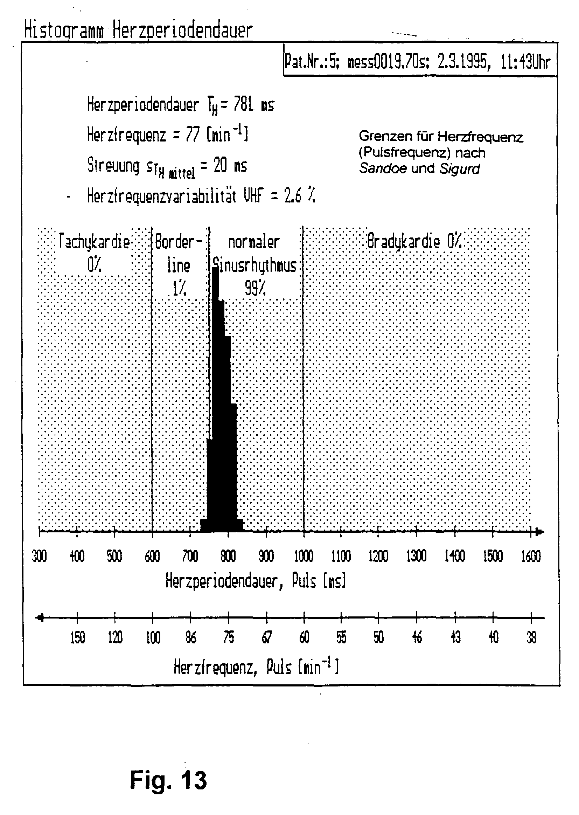

Fig. 13   das aus Fig. 11 abgeleitete Histogramm der Herzperiodendauern einschließlich definierte Grenzen sowie prozentuale Angaben des Auftretens der Perioden in den entsprechenden Bereichen,

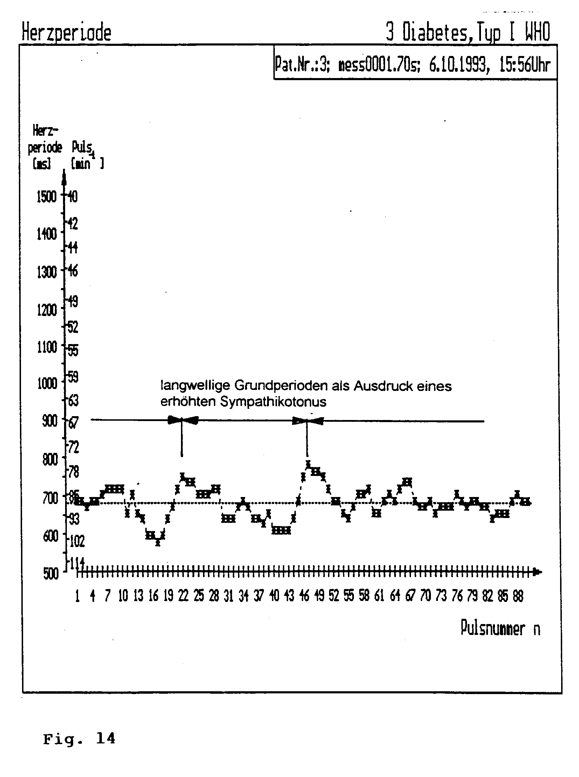

Fig. 14   das Tachogramm $T_H = f(n)$ einer 35jährigen liegenden Typ I-Diabetikerin bei Ruheventilation,

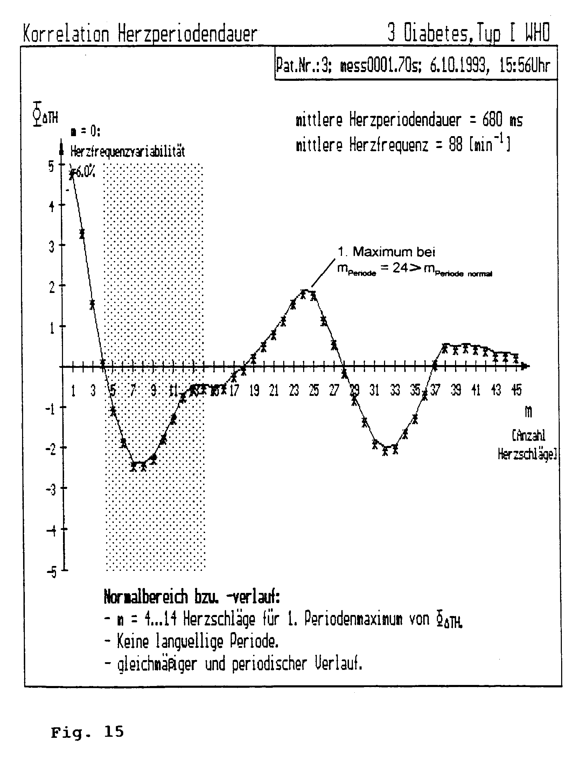

Fig. 15   die zu Fig. 14 gehörende Autokorrelationsfunktion $\Phi_{ATH}(m)$,

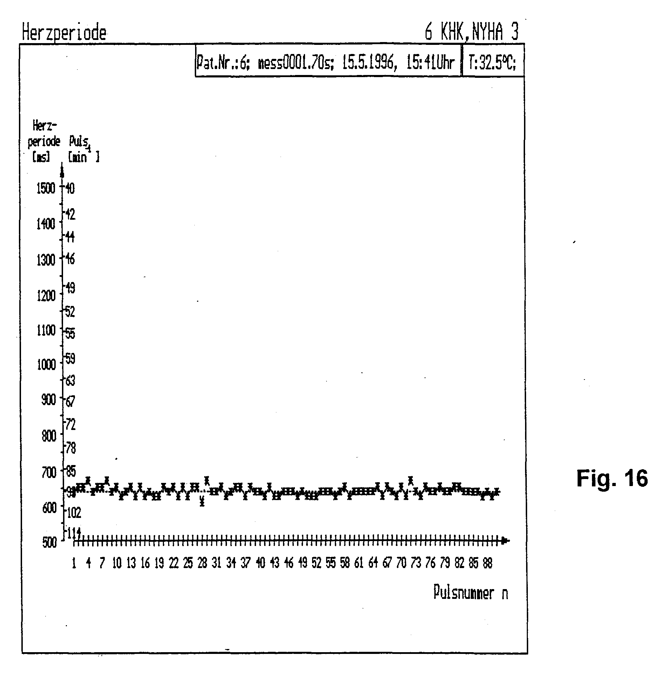

Fig. 16   das Tachogramm $T_H = f(n)$ einer 72jährigen liegenden Patientin mit koronarer 3-Gefäßerkrankung, arterieller Hypertonie und Hinterwandinfarkt bei Ruheatmung,

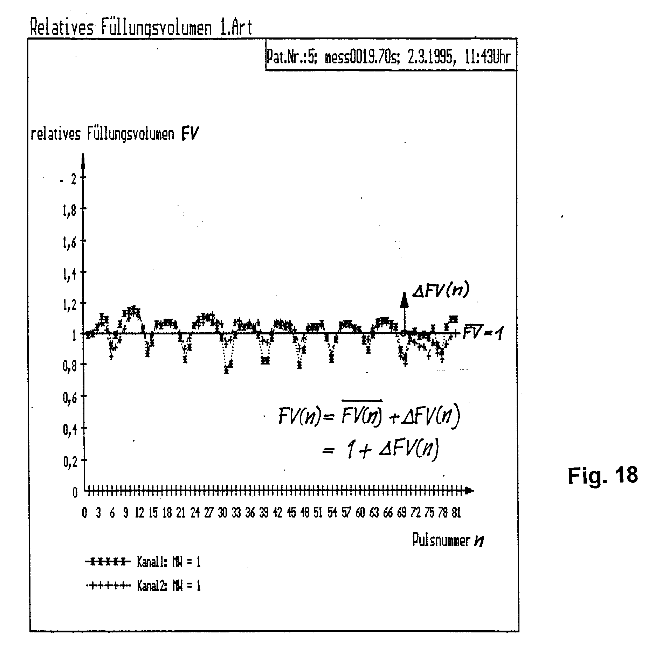

Fig. 18   den Verlauf des relativen Füllungsvolumens (Meßort Fingerbeere) bei einem Normalprobanden,

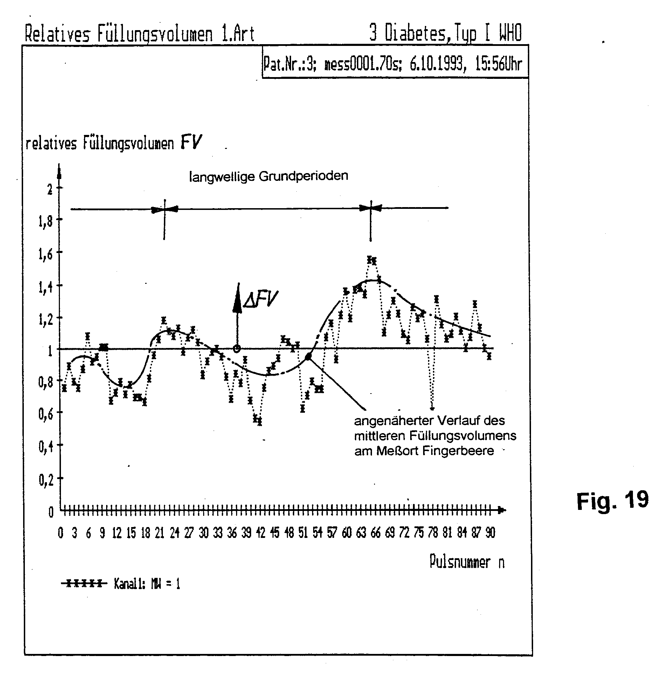

Fig. 19   den Verlauf des relativen Füllungsvolumens (Meßort Fingerberre) bei der Typ I-Diabetikerin nach Fig. 14,

Fig. 20

a) den Verlauf des mikrovaskulären Blutfüllungsvolumens (Kanal 1) bei einem liegenden Normalprobanden und Ruheventilation (Meßort Fingerbeere), wobei wegen übersichtlicherer Darstellung nur das 840nm-Blutfüllungsvolumen abgebildet ist,

b) Verläufe der Füllungsvolumima bei Hyperventilation (Proband atmet sehr tief), wenn a) die Bezugsmessung darstellt,

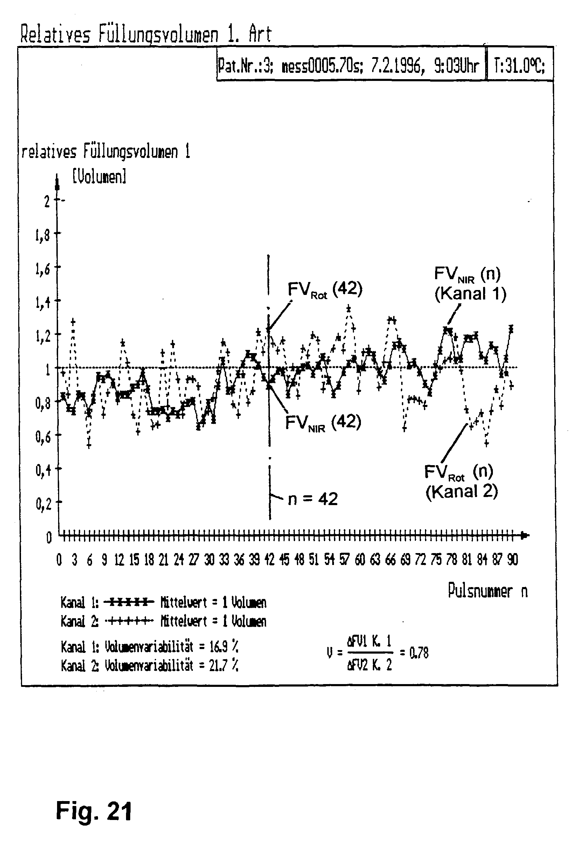

Fig. 21   den Verlauf der Füllungsvolumina 1.Art am Meßort Großzehe bei einer 7jährigen Patientin mit Sichelzellanämie unter Ruheventilation einschließlich Verhältnisse der Füllungsvolumina bei dem Volumenpuls n=42 als Beispiel,

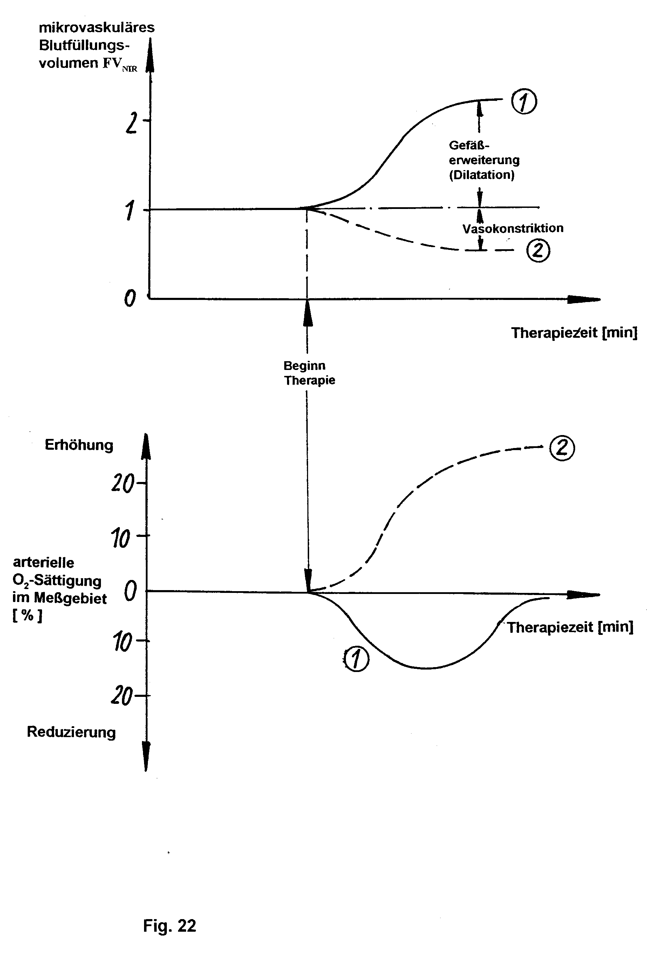

Fig. 22   den qualitativen Verlauf des Blutfüllungsvolumens (Fall der Vasodilatation) bei gleichzeitiger Reduzierung der artiellen $O_2$-Sättigung am Meßort Großzehe in Abhängigkeit der Therapiezeit bei Infusion von Prostavasin sowie den Fall der Erhöhung der arteriellen $O_2$-Sättigung bei auftretender Vasokonstriktion,

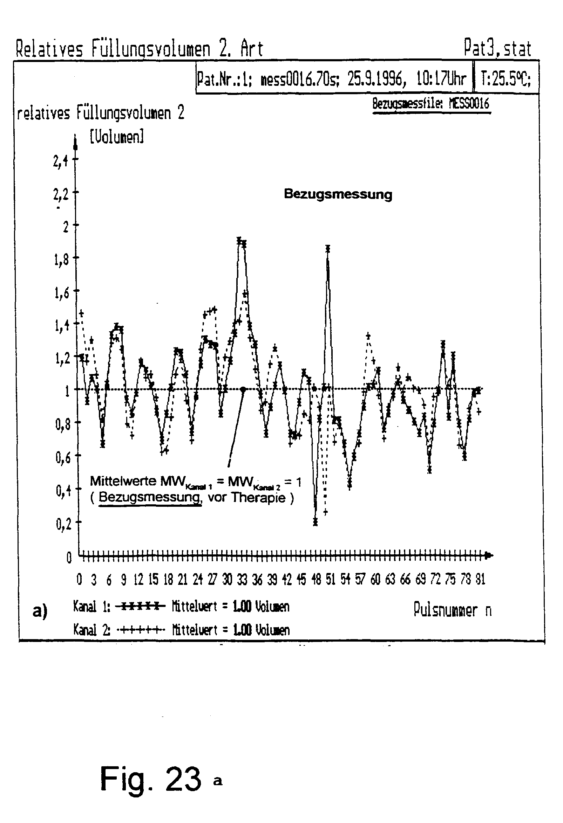

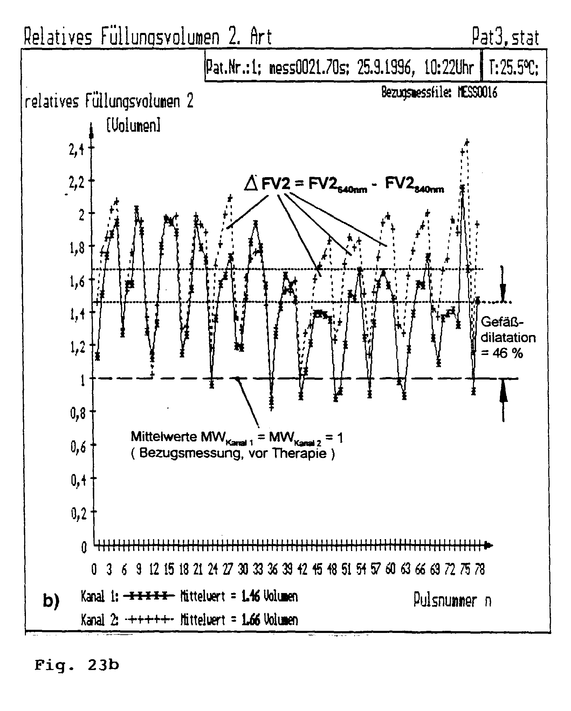

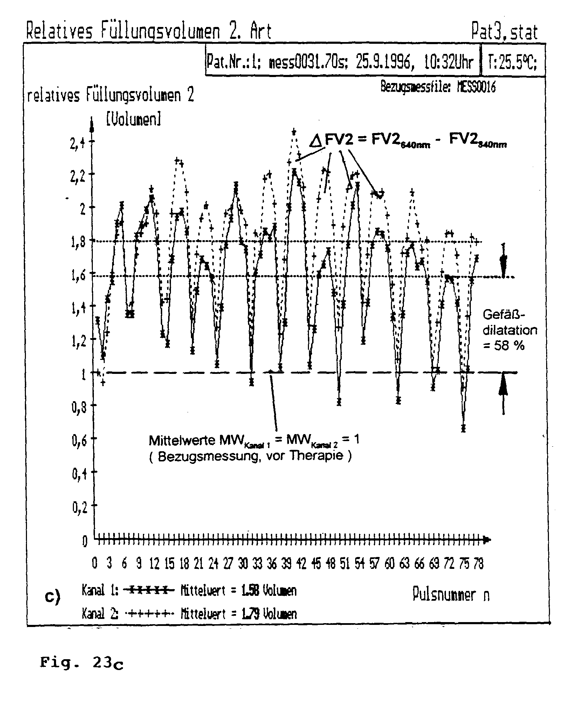

Fig. 23   den Verlauf der relativen Füllungsvolumina 2.Art am NIRP-Meßort Fingerbeere bei hyperbarer Sauerstofftherapie, insbesondere a) vor, b) während und c) am Ende der $O_2$-Maskenatmung (15m Tiefe) bei einem Typ II-Diabetiker mit pAVK und Problemwunde am selbigen Bein und

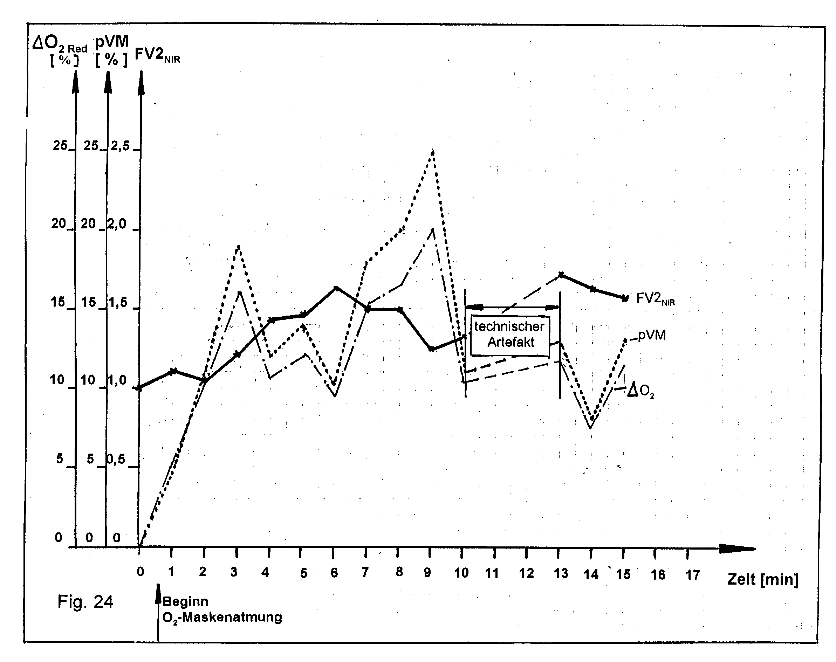

Fig. 24   die bei dem Patienten nach Fig. 23 während der gesamten $O_2$-Maskenatmung ermittelten peripheren Kenngrößen $\Delta O_{2Reduzierung}$, pVM und $FV2_{NIR}$ als Beispiel.

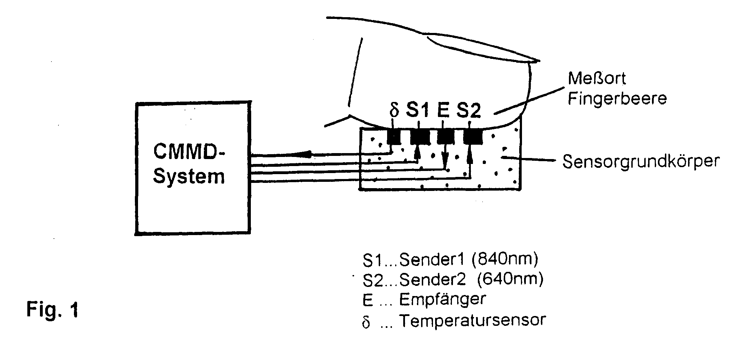

Fig. 1 Zeigt das Meßprinzip der bekannten NIR-ROT-Remissions-Photoplethysmographie (NIRP-Methode) [Christ, F. et al.: Time Discrete, Near Infrared-Photoplethysmography (NIRP) for Non-invasive Investigation of the Volume Pulse in Man. Eur. J. Med. Res. 1, 237-243 (1995/96)].

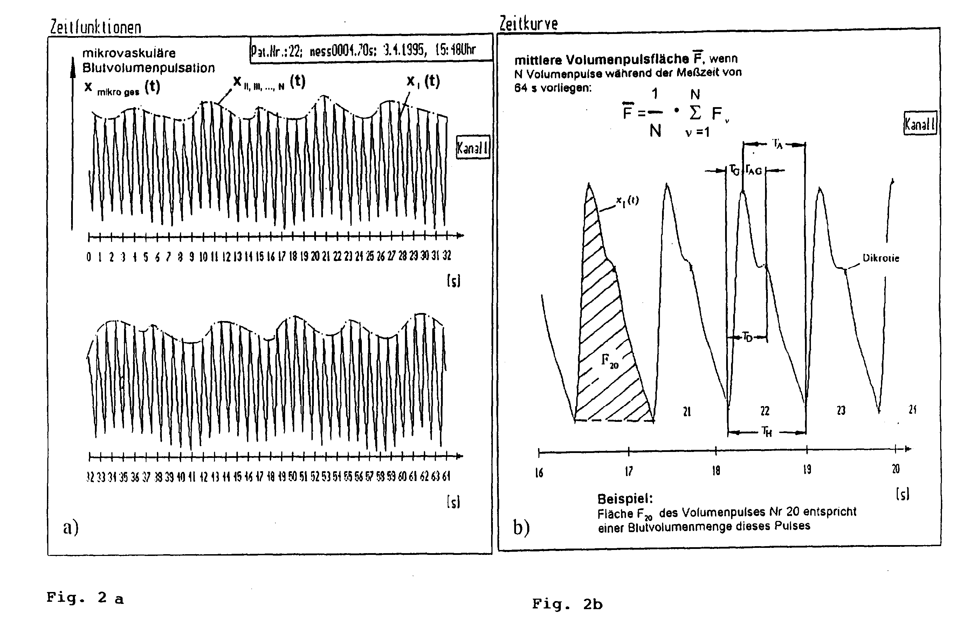

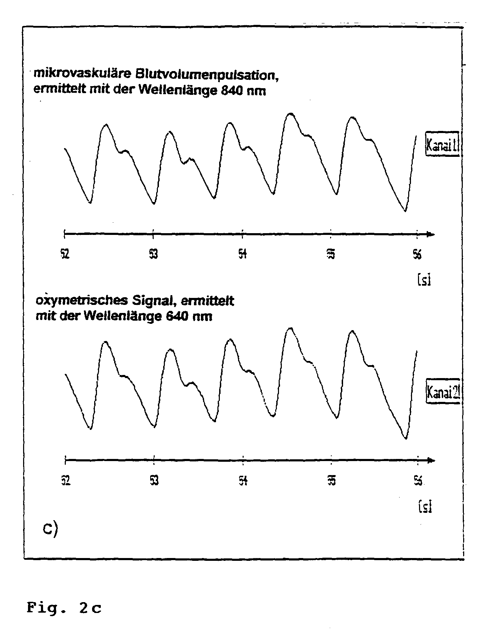

In Fig. 2 sind die Verläufe der mikrovaskulären Blutvolumenpulsation sowie des zugehörenden oxymetrischen Signals bei einem liegenden Normalprobanden (Meßort Fingerbeere) ersichtlich. Neben der Ableitung der Pulsperiode $T_H$ (Herzperiodik) wird die Ermittlung der Füllungsvolumina gezeigt.

Mit der nichtinvasiven NIRP(Nahes-Infrarot-Remissions-Photoplethysmographie)-Methode ist eine Erfassung und Auswertung zentraler und peripherer Herz-Kreislauf-Parameter möglich und damit auch eine Verlaufskontrolle und Therapieüberwachung, einschließlich bei Pharmaka und Drogen. Es wird ein Clip, analog zum bekannten Pulsoxymeter, an einem Peripheren Meßort (z.B. Finger- oder Zehenbeere) angebracht. Zur Diagnostik von Gewebeabschnitten kann die Ableitung der Meßdaten über einen Flächensensor erfolgen. Mit einer in das Gewebe eingestrahlten Wellenlänge von S1=840nm lassen sich remissions-photoplethysmographisch die (relativen) Mengen des Blutes erfassen, die das Mikrogefäßsystem am Meßort in Form von Volumenpulswellen passieren und vom Herzen ausgelöst werden, während mit der (im Zeitmultiplex) eingestrahlten roten Wellenlänge von S2=640nm eine Bewertung des oxymetrischen Zustandes am Meßort möglich ist. Zur Kontrolle der Temperatur am Meßort ist ein entsprechender Sensor im Clip integriert (Fig. 1).

Die bereits erwähnte Fig. 2 zeigt eine gemessene Blutvolumenpulsation im Mikrogefäßbereich "Fingerbeere" bei einem liegenden Normalprobanden mit Ruheventilation (Meßzeit 64s), dabei sowohl in einem 4s-Zeitfenster (mit den charakteristischen Zeitparametern und der eingezeichneten Volumenpulsfläche als quantitatives Maß für das mikrovaskuläre Blutfüllungsvolumen) als auch zusammen mit dem oxymetrischen 640nm-Signal. Es ist ersichtlich, daß ebenso eine Pulsperiodendauer je Herzaktion abgeleitet werden kann, die am Meßort Fingerbeere mit ausreichender Genauigkeit mit der Herzperiodendauer $T_H$ gleichgesetzt werden kann, auch wenn zwischen der R-Zacke im EKG und dem Pulskurvenminimum eine Laufzeit auftritt. Die erzwungene Schwingung "Herzpulsation" ist an der Fingerbeere meßbar.

Ohne Amplituden-Kalibrierung ist bei nichtinvasiven Verfahren die Messung einer absoluten Mikrogefäß-Durchflußmenge (z.B. in ml) nicht möglich. Deshalb wird aus den Pulsflächen, wie aus Fig. 2 hervorgeht, ein "relatives mikrovaskuläres Blutfüllungsvolumen FV" als ein peripherer Kreislauf-Parameter abgeleitet, da die Volumenpulsflächen den (relativen) Blutvolumenmengen entsprechen. Zwecks Normierung wird hierbei die Fläche eines jeden Volumenpulses einer Messung durch den berechneten mittleren Flacheninhalt $\overline{F}$ dividiert. Also beträgt in Fig. 2 die Größe des sich einstellenden (dimensionslosen, relativen) mikrovaskulären Blutfüllungsvolumens FV bei z.B. Pulsnummer n = 20

$$FV(20) = \frac{F_{20}}{\overline{F}} \; .$$

Der Mittelwert aller Beträge von FV ist demnach für die zugrunde gelegte Meßzeit (z.B. 64s) wegen der vorgenommenen Normierung immer gleich Eins und liefert eigentlich für die Bewertung keine Information, wohl aber die sich um diesen Mittelwert $\overline{FV} = 1$ einstellenden Blutvolumenänderungen bei den einzelnen Volumenpulsen n.
Für den Normalfall ist folgender Verlauf charakteristisch:

\* Es bildet sich bei diesen relativen mikrovaskulären Blutfüllungsvolumina die Atmungsperiodik ab, ausgedrückt durch ein Vielfaches einer Pulsnummer (bzw. einer Herzaktion).

\* Als Maß für die Abweichung vom Mittelwert $\overline{FV}=1$ läßt sich die bekannte Standardabweichung, die Variabilität $\Delta FV$ des Blutfüllungsvolumens, bestimmen.

Die Ermittlung der Füllungsvolumina wird sowohl für das Blutvolumensignal als auch das oxymetrische Signal vorgenommen. Dabei sind bei normaler Oxygenierung beide Verläufe nahezu deckungsgleich.

Sollen die Füllungsvolumina bei angelegtem Sensor unterschiedlicher Messungen, also bei verschiedenen Zeitpunkten (auch von Ruhe- und Hyperventilation), verglichen werden, so läßt sich durch Division der Volumenpulsflächen der aktuellen Messung und dem Pulsflächen-Mittelwert einer Bezugsmessung die im Mittel auftretende Veränderung sowohl vom Blutvolumensignal als auch oxymetrischen Signal darstellen. Es wird dann vom "relativen Füllungsvolumen 2.Art" gesprochen, während das Füllungsvolumen 1.Art immer den Mittelwert $\overline{FV}=1$ besitzt, wie oben gezeigt wurde.

Aus der bisherigen zusammenfassenden Darstellung sollen die weiteren Parameter und Kennfunktionen wie folgt abgeleitet werden.

peripherer Mikrozirkulations-Koeffizient pMC:

Es ist das "Ähnlichkeitsmaß" zwischen dem mikrovaskulären Blutvolumensignal und dem oxymetrischen Signal bzw. der Korrelationsfaktor zwischen beiden Signalen. pMC läßt sich bei Periodischen Signalen je Herzaktion bestimmen. Bei nichtperiodischen Signalen werden die Signale in 1s-Intervalle zerlegt, hierfür jeweils der pMC-Wert ermittelt, daraus Mittelwert und Streuung.

Allgemein ermöglicht pMC eine quantitative Beurteilung des Zusammenwirkens von Mikrogefäßen, Blutfluß und vasomotorischer Fasern peripherer Nerven.

periphere Volumen-Koeffizienten pVC:

Es werden Relationen zwischen den Füllungsvolumina 1.Art von $FV1_{NIR}(n)$ und $FV1_{ROT}(n)$ ermittelt.

$pVC_1$: stellt die nomierte Kovarianz r zwischen beiden dar.
$pVC_2$: ergibt sich aus dem Produkt-Mittelwert aus beiden Verläufen.
$pVC_3$: ist der Quotient-Mittelwert.

peripheres Vasodilatations-Maß pVM:

Es ist ein Maß für maximale Gefäßerweiterung durch Medikamenteneinwirkung bei nur geringer $O_2$-Reduzierung am Meßort, d.h. pVM soll minimal werden.

Veränderung der arteriellen O2-Sättigung $\Delta O_2$ am Meßort:

Wird abgeleitet aus den relativen Füllungsvolumina 2.Art als Differenz dieser Füllungsvolumina, bezogen auf FV2 des oxymetrischen Signals.

Veränderung des peripheren Strömungswiderstandes $\Delta PW$:

Da der periphere Strömungswiderstand (bei Konstanz des Druckes) umso kleiner ist, desto größer das mikrovaskuläre Blutfüllungsvolumen und desto kleiner die Pulsperiodik sich erweisen, dabei aber kein Absolutwert für PW ermittelt werden soll, wird die Veränderung des peripheren Strömungswiderstandes als relatives Maß der Verhältnisse von Pulsperiodik $FV2_{NIR}$ von aktueller Messung zur Bezugsmessung definiert.

arteriolo-venoläre $O_2$-Differenz:

Wird im Sinne der Signaltheorie als Abstandsmaß d zweier Signale abgeleitet und definiert:

$$d = \sqrt{1-[pMC]^2} \ .$$

Tachogramme:

Stellen die Abhängigkeit der ermittelten Parameter $T_H$, FV1, pMC, d, pVM; $pVC_{2,3}$, $\Delta O_2$ von der jeweiligen Herzaktion n dar, woraus Variabilitäten und mittlere Perioden (als Vielfache m einer Herzaktion) in Form einer verallgemeinerten Mittelwertfunktion [Autokorrelationsfunktion Ø(m)] ableitbar sind.

Oxygenierung:

Reversible koordinative Bindung von Sauerstoff an das zweiwertige Eisen des Hämoglobins, wobei das desoxygenierte Hämoglobin in das oxygenierte umgewandelt wird. Die Abspaltung von $O_2$ wird als Desoxygenation bezeichnet.

Fig. 3 zeigt den Verlauf des Abstandsmaßes d der Photoplethysmographie-Signale $x_1(t) = x_{NIR}(t)$, $x_2(t) = x_{ROT}(t)$ bzw. der relativen arteriolo-venolären Differenz $avdO_2^*$ der $O_2$-Sättigung des Blutes in Abhängigkeit des entsprechenden Korrelationsfaktors bzw. des definierten peripheren Mikrozirkulationskoeffizienten pMC. Ebenfalls sind eingetragen die ermittelten Normalbereiche. Es ist ersichtlich, daß bei pMC-Werten (Korrelationsfaktoren) gegen Eins die relative arteriolo-venoläre Differenz der $O_2$-Sättigung des Blutes nach Null verläuft und umgekehrt.

Fig. 4 zeigt den pMC-Verlauf bei einem liegenden Normalprobanden bei Ruheventilation einschließlich Mittelwert $\overline{pMC}$ sowie $\Delta pMC$ als jeweilige Abweichung. Die ermittelte Variabilität entspricht der Standardabweichung. Deutlich zeigt sich die Wirkung der respiratorischen Arrhythmie mit Zunahme von pMC im Meßgebiet während der Inspirationsphase sowie Rückgang während Exspirationsphase der Sinusarrhythmie.

Fig. 5 zeigt Verläufe von pMC am Meßort Großzehe a) vor und b) nach einer Infusion von Prostavasin bei einem 72jährigen Patienten mit peripherer arterieller Verschlußkrankheit (pAVK). Neben einer Erhöhung des mittleren pMC-Grades durch diese Infusion wird die Verringerung der pMC-Variabilität deutlich.

Aus Fig. 6 gehen die pMC-Verhältnisse mit z.T. atmungsdominanten Perioden hoher Variablilität bei einem liegenden Patienten mit Linksherzinsuffizienz hervor.

Fig. 7 zeigt die Verhältnisse von pMC bei einem liegenden Patienten mit diabetischer Polyneuropathie (Meßort Großzehe). Wegen der im Normalfall bei Hyperventilation HV ausgelösten akralen Vasokonstriktion verringert sich im Normalfall der Mittelwert von pMC im Vergleich zu dem bei Ruheventilation und erhöht sich dessen Variabilität. Im vorliegenden Fall nach Fig. 7 erhöht sich bei dem Funktionstest HV der Mittelwert von pMC und verringert sich die Variabilität, was typisch ist für eine (diabetische) Polyneuropathie. Unter HV erhöht sich des weiteren das Blutvolumen um 12%, d.h. es ist eine Öffnung der arterio-venösen Shunts am Meßort eingetreten. Somit ermöglicht der Funktionstest HV auch eine Prüfung der Öffnung dieser Shunts.

Fig. 8 zeigt die Verläufe der mit NIR-ROT-Remissions-Photoplethysmographie gemessenen integralen Mikrozirkulation am Meßort Großzehe bei einem 85jährigen Patienten mit pAVK. Da kaum von periodischen Volumenpulsationen gesprochen werden kann, wird bei solchen Verläufen der Zeitfunktionen der Mittelwert von pMC (Korrelationsfaktor)

$$\overline{pMC} = \frac{\overline{x_{NIR}(t) \cdot x_{ROT}(t)}}{\sqrt{\overline{x^2_{NIR}(t)} \cdot \overline{x^2_{ROT}(t)}}}$$

einschließlich Variabilität derart bestimmt, daß die erhaltenen Verläufe im Kanal 1 und 2 in Sekunden-Intervalle zerlegt werden, hierfür der jeweilige pMC-Wert bestimmt wird und damit für die gesamte Meßzeit (z.B. 64s) $\overline{pMC}$ sowie die pMC-Variabilität ermittelt werden. Die in Fig. 8 abgeleiteten pMC-Werte sind nicht normal.

In Fig. 9 sind die bei einem Normalprobanden ermittelten Remissions-Photoplethysmographie-Signale bei Lungenatmung mit überlagerten Volumenpulsationen am Meßort Oberkörper (Lungennahe) dargestellt. Der während einer Atmungsperiode $T_{Atmung}$ sich einstellende pMC-Grad $pMC(n^*)_{Lunge}$ wird analog zu pMC(n) bestimmt. Zur Unterdrückung der höherfrequenten Volumenpulsationen im NIR- und ROT-Signal wird jeweils der gleiche Tiefpaß mit einer Grenzfrequenz von ca. 0,6 Hz diesen Signalen nachgeschaltet, um nachfolgend den pMC-Verlauf $PMC(n^*)_{Lunge}$ zu ermitteln.

Mit der Nahen-Infrarot-Remissions-Photoplethysmographie läßt sich, wie bereits vorn dargestellt wurde, nichtinvasiv an einem entsprechenden peripheren Ort (z.B. Fingerbeere) die relative Menge des Blutes je Herzaktion bei einer ausgewählten Meßzeit erfassen, die das Mikrogefäßsystem in Form von Volumenpulswellen passiert.

Da die Volumenpulsation an einer "Endstelle" des arteriellen Gefäßsystems gemessen wird, enthält sie bekanntlich alle wesentlichen Parameter, die das Zusammenwirken der "Zentrale", dem Herzen, mit dem dazwischen liegenden Gefäßsystem charakterisieren:

-    Herzperiodendauer $T_H$ und deren Veränderung je Herzpulsation während der Meßzeit (z.B. 64 s). Es kann angenommen werden, daß im Normalfall (keine pAVK auf der "Übertragungsstrecke", keine Stenosen) die dort gemessene Pulsperiodendauer mit der Herzperiodendauer $T_H$ mit ausreichender Näherung gleichgesetzt werden kann. Als Meßort ist deshalb im allgemeinen die Fingerbeere zu wählen, falls dort keine Durchblutungsstörungen auftreten (Cave: niedrigerer Blutdruck auf der Seite der Messung).

-    Parameter des Mikro- und arteriellen Makrogefäßbereiches, (u.a. Zeitparameter des NIR-Volumenpulses, das mikrovaskuläre Blutfüllungsvolumen einschließlich oxymetrisches Füllungsvolumen, Änderung der Oxygenierung, abgeleitete periphere Kennfunktionen pMC, pVC, PVM).

Hierzu sei auch auf die Veröffentlichungen "Perfusion" 7/96, 9. Jahrgang, Seite 268 bis 279, "Perfusion" 8/96, 9. Jahrg., Seiten 300 bis 310, sowie "European Journal of Medical Research", Vol. 1, 22. Februar 1996, Seiten 237 bis 244 verwiesen, die zur Erläuterung des hier vorgestellten Gegenstandes als zugehörig betrachtet werden.

Fig. 10a zeigt den Zeitausschnitt einer an einer Fingerbeere gemessenen NIR-Blutvolumenpulsation einschließlich markierte Herzperiodendauern [$T_H(n)$ zum n-ten Volumenpuls gehörend usw.] sowie Fig. 10b das daraus abgeleitete Tachogramm $T_H(n)$ als Beispiel. Ersichtlich ist das Auftreten von Herzperiodendauerabweichungen $\Delta T_H(n)$ vom Mittelwert $\overline{T_H(n)}$ sowie eine sich einstellende Herzfrequenzvariabilität.

In Fig. 11 ist das Tachogramm $T_H = f(n)$ bei einem 40jährigen Normalprobanden dargestellt, wo die respiratorische Sinusarrhythmie deutlich wird, während Fig. 12 die zur Herzperiodendauerabweichung $\Delta T_H(n)$ zugehörige Auto-

korrelationsfunktion $\Phi_{\Delta TH}(m)$ als Mittelwertfunktion einschließlich Normalbereich sowie in $\Phi_{\Delta TH}(m)$ auftretende Kennwerte (u.a. respiratorische Sinusarrhythmie mit 8 Herzschlägen im Mittel) wiedergibt.

Fig. 13 zeigt das für den Normalprobanden nach Fig. 11 sich ergebende Histogramm der Herzperiodendauer einschließlich definierte Grenzen mit prozentualen Angaben des Auftretens der Perioden in den entsprechenden Bereichen.

Die Fig. 14 und 15 geben die kardiovaskulären Kennfunktionen bei einer 35jährigen Diabetikerin (Typ I WHO) wieder. Das Auftreten von anormalen langen Grundperioden als Ausdruck eines erhöhten Sympathikotonus bzw. einer veränderten Herzmikrozirkulation ist deutlich.

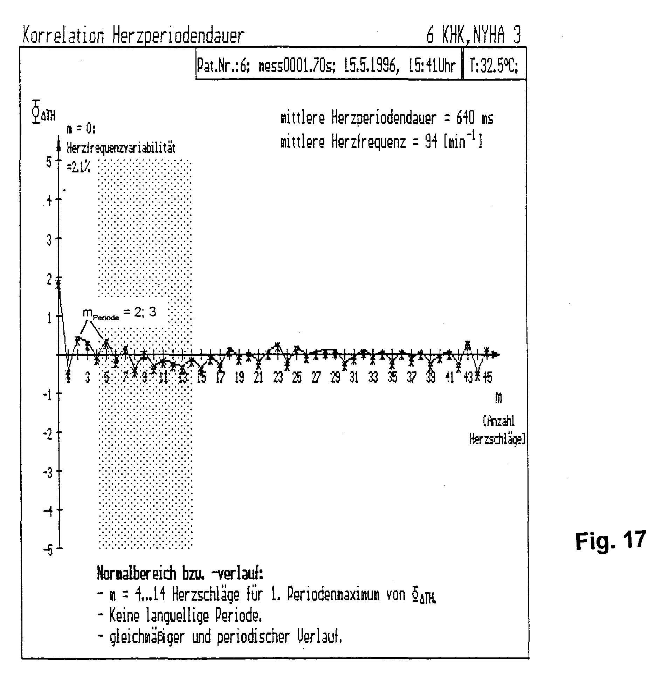

Die Fig. 16 und 17 zeigen die Verläufe bei einer 72jährigen liegenden Patientin bei Ruheatmung mit koronarer 3-Gefäßerkrankung, arterieller Hypertonie und Hinterwandinfarkt. Die Unterschiede zum Normalprobanden nach den Fig. 11, 12 werden ebenso deutlich wie zur Typ I-Diabetikerin nach den Fig. 14, 15. Charakteristisch für den Verlauf nach Fig. 17 ist die geringe Herzfrequenzvariabilität, insbesondere das Auftreten von Perioden von 2 bzw. 3 Herzschlägen.

Als "relatives (mikrovaskuläres) Füllungsvolumen" an einer entsprechenden Meßstelle (einem Mikrogefäßbereich) wird die Fläche eines jeden Volumenpulses, bezogen auf den mittleren Flächeninhalt aller Pulsationen in einem Meßfile (z.B. 80 Volumenpulse) ermittelt, wie aus Fig. 2b hervorgeht.

Fig. 18 zeigt den Verlauf eines solchen relativen Füllungsvolumens (Meßstelle 2. Fingerbeere re.) bei dem Normalprobanden und bei Ruheventilation nach Fig. 11, Fig. 19 den für den Diabetiker nach Fig. 14 (Meßstelle 2. Fingerbeere re.).

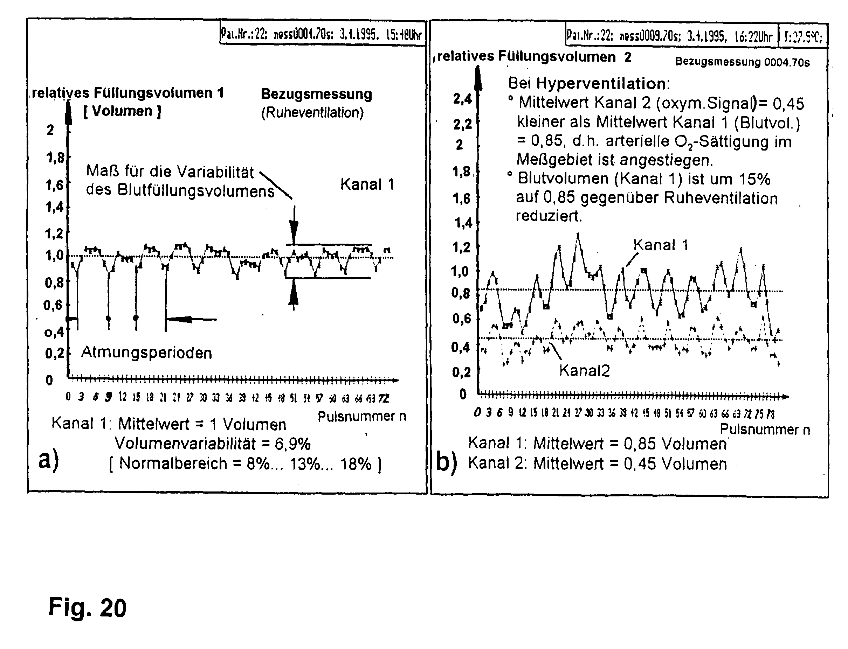

In Fig. 20 sind die Verläufe der Füllungsvolumina bei einem Probanden mit Ruhe- und Hyperventilation dargestellt. Während sich bei Ruheventilation die Atmungsdominanz sowie die Füllungsvolumen-Variabilität beim mikrovaskulären Blutfüllungsvolumen deutlich abbilden, reduziert sich bei Hyperventilation das NIR-Füllungsvolumen im Kanal 1 durch Vasokonstriktion um 15%, die arterielle $O_2$-Sättigung nimmt an der Meßstelle extrem zu.

Fig. 21 zeigt den Verlauf der Füllungsvolumina am Meßort Großzehe unter Ruheventilation bei einem Patienten mit Sichelzellanämie. Neben den unterschiedlichen Variabilitäten der NIR- und ROT-Füllungsvolumina ist offensichtlich, daß beide Verläufe nicht deckungsgleich sind. Die Ermittlung der peripheren Volumen-Koeffizienten $pVC_{1,2}$ zeigt eine beträchtliche Reduzierung zum Normalfall.

In Fig. 22 sind die qualitativen Verläufe vor und während einer Infusion von Prostavasin (Meßort Großzehe) bei Patienten mit pAVK ersichtlich. Während im 1. Fall eine Gefäßdilatation (Erhöhung des mikrovaskulären Blutfüllungsvolumens als das Ziel der Therapie) in Verbindung mit einer Reduzierung der arteriellen Sauerstoffsättigung im Meßgebiet auftritt, liegt im Fall 2 eine Vasokonstriktion in Korrelation zu einer Erhöhung der arteriellen $O_2$-Sättigung am Meßort vor.

Fig. 23 zeigt die Verhältnisse bei hyperbarer Sauerstoff-Therapie, dabei Fig. 23a) vor, b) während und c) am Ende einer $O_2$-Maskenatmung (entsprechend in 15m Tiefe). Wie schon in Fig. 22 ersichtlich, tritt eine Gefäßdilatation während der Therapie auf und reduziert sich die arterielle $O_2$-Sättigung am Meßort deutlich ($\Delta FV2 = FV2_{640nm} - FV2_{840nm} > 0$). Ermittelt man für die dargestellten Zeitpunkte der hyperbaren Sauerstoff-Therapie bei diesem Patienten die im Vergleich zur Bezugsmessung a) veränderten peripheren Strömungswiderstände am Meßort, so erhält man Reduzierungen auf 71% bei b) sowie auf 66% bei c).

In Fig. 24 sind die arterielle $O_2$-Reduzierung, das periphere Vasodilatations-Maß pVM sowie das mikrovaskuläre Blutfüllungsvolumen $FV2_{NIR}$ während der gesamten $O_2$-Maskenatmung nach Fig. 23 ersichtlich. Deutlich bilden sich auftretende Perioden ab. Ebenfalls erkennt man bei diesem Typ II-Diabetiker, daß das periphere Vasodilatationsmaß kaum vom Verlauf von $FV2_{NIR}$ abhängig ist, jeoch stark korreliert mit der arteriellen $O_2$-Reduzierung am Meßort.

## Patentansprüche

1. Vorrichtung zur nichtinvasiven Messung und Auswertung von Kreislauf-Kennfunktionen und -Parametern insbesondere des Blutgefäßsystems einschließlich des Herzens und der Lunge im Zusammenwirken mit dem autonomen Nervensystem,
   gekennzeichnet durch

   - Mittel zum Erfassen von Parametern zur Ableitung von Zustandsfunktionen durch nichtinvasive optische und/oder elektrische Messung,
   - Zustandsbeschreibung je Herzaktion und daraus abgeleiteter Auto- und/oder Kreuzkorrelationsfunktionen als verallgemeinerte Mittelwertfunktionen bzw. zugehöriger Amplitudenspektren als Zustandsfunktionen,
   - Mittel zum Speichern und Darstellen der Zustandsfunktionen und
   - Mittel zum Vergleichen der erhaltenen Zustandsfunktionen mit Normalwerten und -verläufen.

2. Vorrichtung nach Anspruch 1,

dadurch gekennzeichnet,
daß auf der Basis einer Zwei-Wellenlängen-NIR-ROT-Remissions-Photoplethysmographie und der daraus folgenden Zeitfunktionen

$$x_{840nm}(t) = X_{NIR}(t) = x_1(t) \quad \text{[mikrovaskuläre Blutvolumenpulsation]}$$

$$x_{640nm}(t) = x_{ROT}(t) = x_2(t) \quad \text{[oxymetrisches Signal]}$$

Mittel zur Bestimmung von Kreislauf-Kennfunktionen je Herzaktion n vorgesehen sind, insbesondere für

- peripherer Mikrozirkulations-Koffizient $\overline{pMC(n)}$ als das Ähnlichkeitsmaß bzw. Korrelationsfaktor dieser Funktionen

$$r = \frac{\overline{x_1(t)\ x_2(t)}}{\sqrt{\overline{x_1^2(t)} \cdot \overline{x_2^2(t)}}} \quad \text{bzw.} \quad \overset{\wedge}{r}(n) = pMC(n) = \frac{\overline{x_{NIR}(n) \cdot x_{ROT}(n)}}{\sqrt{\overline{x_{NIR}^2(n)} \cdot \overline{x_{ROT}^2(n)}}}$$

- arteriolo-venoläre Sauerstoff-Differenz $avdO_2{}^*$ als Abstandsmaß d der Funktionen $x_{NIR}(n)$, $x_{ROT}(n)$:

$$avdO_2{}^*(n) \triangleq d(n) = \sqrt{1 - [pMC(n)]^2} \,,$$

- peripherer Volumen-Koeffizient $\overline{pVC_1}$ als Ähnlichkeitsmaß (normierte Kovarianz r) der mikrovaskulären und
oxymetrischen Füllungsvolumina $FV1_{NIR}(n)$ und $FV1_{ROT}(n)$

$$\overline{pVC}_1 \triangleq r_{FV1\ NIR,\ ROT} \,,$$

- peripherer Volumen-Koeffizient $\overline{pCV_2(n)}$ als Produkt-Mittelwert der Füllungsvolumina

$$\overline{pVC_2} = \frac{1}{N} \sum_{n=1}^{N} FV1_{NIR}(n) \cdot FV1_{ROT}(n) \,,$$

- peripherer Volumen-Koffizient $pVC_3(n)$ als Quotient-Mittelwert der Füllungsvolumina

$$\overline{pVC_3} = \frac{1}{N} \sum_{n=1}^{N} \frac{FV1_{NIR}(n)}{FV1_{ROT}(n)} \,,$$

- prozentuale arterielle Sauerstoff-Sättigungs-Veränderung im Vergleich zu einer Bezugsmessung bei der damit
möglichen Ermittlung eines Füllungsvolumens 2. Art $FV2_{NIR,\ ROT}$

$$\Delta O_{2Reduzierung}(n) \ [\%] \ = \ 100 \ \frac{FV2_{ROT}(n) - FV2_{NIR}(n)}{FV2_{ROT}(n)} \ [\%] \ ,$$

$$\Delta O_{2Erh\ddot{o}hung}(n) \ [\%] \ = \ 100 \ \frac{FV2_{NIR}(n) - FV2_{ROT}(n)}{FV2_{ROT}(n)} \ [\%] \ ,$$

- peripheres Vasodilatations-Maß pVM als Güte einer auftretenden Vasodilatation, d.h. einer möglichst großen Gefäßerweiterung bei geringer $O_2$-Reduzierung am Meßort:

$$pVM(n) \ = \ \frac{FV2_{ROT}(n) \ - \ FV2_{NIR}(n)}{FV2_{NIR}(n)} \ \rightarrow \ Minimum$$

3. Arbeitsverfahren zum Betreiben der Vorrichtung nach Anspruch 1 oder 2,
   dadurch gekennzeichnet,
   daß aus den ungestörten Volumenpulsationen des NIR-Signals bei Transmissions- oder Remissionsphotoplethysmographie sowie bei der elektronischen Oszillographie bei einer vorgegebenen Meßzeit das Herzperiodendauer-Tachogramm $T_H = f(n)$, d.h. je Herzaktion n, abgeleitet und dargestellt wird, um Perioden und Abweichungen vom Mittelwert $\overline{T_H(n)}$ zu bestimmen.

4. Arbeitsverfahren nach Anspruch 3,
   dadurch gekennzeichnet,
   daß aus der Abweichung der Herzperiodendauer vom linearen Mittelwert je Herzaktion n

$$\Delta T_H(n) = T_H(n) - \overline{T_H(n)}$$

   deren Autokorrelationsfunktion

$$\Phi_{\Delta TH}(m) = \overline{\Delta T_H(n) \cdot \Delta T_H(n-m)}$$

   gebildet wird, um daraus die in der Zeitreihe $T_H = f(n)$ auftretenden mittleren Perioden m als Vielfaches der Herzfrequenz darzustellen, und die bei m=0 auftretende Standardabweichung $S_{TH}$ als mittlere quadratische Abweichung der Herzperiodendauer bzw. durch Bezug auf den linearen Mittelwert $\overline{T_H(n)}$ die Herzfrequenzvariabilität VHF

$$VHF \ = \ \frac{S_{TH}}{\overline{T_H(n)}} \ \cdot \ 100 \ [\%] \ \ zu \ ermitteln.$$

5. Arbeitsverfahren nach Anspruch 4,
   dadurch gekennzeichnet,
   daß im Bildungsalgorithmus von $\Phi_{\Delta TH}(m)$ im Tachogramm $T_H = f(n)$ auftretende Extrasystolen durch den Mittel-wert $\overline{T_H(n)}$ ersetzt werden.

6. Arbeitsverfahren nach Anspruch 4,

dadurch gekennzeichnet,
daß bei Vorliegen von Perfusionsgleichgewicht der erhaltene Verlauf $\Phi_{\Delta TH}(m)$ mit Normalverläufen ($m_{Periode\ normal}$ = 4...14 Herzschläge) verglichen wird.

7.  Arbeitsverfahren nach den Ansprüchen 4 oder 6,
    dadurch gekennzeichnet,
    daß in $\Phi_{\Delta TH}(m)$ auftretende Perioden ($m_{Periode}$ = 2;3; >14 Herzschläge) Nichtnormalverläufe darstellen.

8.  Arbeitsverfahren nach einem der Ansprüche 4, 6 oder 7,
    dadurch gekennzeichnet,
    daß zur Charakterisierung des kardiovaskulären Zustandes neben der Zustandsfunktion $\Phi_{\Delta TH}(m)$ die weiteren Kennwerte bzw. -funktionen systolischer und diastolischer Blutdruck, pMC-Verlauf einschließlich dessen Autokorrelationsfunktion $\Phi_{\Delta pMC}(m)$ und/oder relatives Füllungsvolumen FV einschließlich dessen Autokorrelationsfunktion $\Phi_{\Delta FV}(m)$ Verwendung finden.

9.  Arbeitsverfahren nach den Ansprüchen 3 oder 4,
    dadurch gekennzeichnet,
    daß als bezogene Funktionstests des kardiovaskulären Systems die tiefe Atmung zur Erhöhung der Förderleistung des Herzens sowie die reflektorische Vasokonstriktion durch Sympathikusreizung benutzt werden und sich dadurch im Normalfall Veränderungen der Kennfunktionen $T_H = f(n)$, $\Phi_{\Delta TH}(m)$ ergeben, die zur Bewertung heranziehbar sind.

10. Arbeitsverfahren nach Anspruch 4,
    dadurch gekennzeichnet,
    daß die Autokorrelationsfunktion $\Phi_{\Delta TH}(m)$ aus dem RR-Abstand im EKG gebildet wird.

11. Arbeitsverfahren nach den Ansprüchen 3 oder 4,
    dadurch gekennzeichnet,
    daß durch Fouriertransformation von $\Phi_{\Delta TH}(m)$ das gleichwertige Leistungsspektrum der Herzfrequenzvariabilität gebildet und als Vergleichswert zur Auswertung herangezogen wird.

12. Arbeitsverfahren nach einem der Ansprüche 3 bis 11,
    dadurch gekennzeichnet,
    daß aus dem Tachogramm des relativen Füllungsvolumens FV(n) die Autokorrelationsfunktion als Zustandsfunktion

$$\Phi_{\Delta FV}(m) = \overline{FV(n) \cdot FV(n\text{-}m)} - 1$$

ermittelt wird, woraus bei m=0 die "Füllungsvolumenvariabilität" FVV sowie aus $\Phi_{\Delta FV}(m)$ die auftretenden mittleren Perioden ableitbar sind.

13. Arbeitsverfahren nach Anspruch 12,
    dadurch gekennzeichnet,
    daß bei Vorliegen von Perfusionsgleichgewicht der erhaltene Verlauf $\Phi_{\Delta FV}(m)$ mit Normalverläufen verglichen und zur Auswertung herangezogen wird.

14. Arbeitsverfahren nach Anspruch 12,
    dadurch gekennzeichnet,
    daß bei Hyperventilation Veränderungen von FV(n) sowie von $\Phi_{\Delta FV}(m)$ im Vergleich zur Ruheventilation hervorgerufen werden, die quantitativ aus diesen Kennfunktionen ermittelbar sind.

15. Arbeitsverfahren nach den Ansprüchen 3 oder 4,
    dadurch gekennzeichnet,
    daß aus dem Tachogramm pMC = f(n) die Autokorrelationsfunktion

$$\Phi_{\Delta pMC}(m) = \overline{pMC(n) \cdot pMC(n\text{-}m)} - [\overline{pMC(n)}]^2$$

als Zustandsfunktion ermittelt wird, woraus bei m = 0 die pMC-Variabilität pMC V sowie aus $\Phi_{\Delta pMC}(m)$ die auftre-

tenden mittleren Perioden abgeleitet und zur Auswertung herangezogen werden.

16. Arbeitsverfahren nach Anspruch 15,
dadurch gekennzeichnet,
daß bei Vorliegen von Perfusionsgleichgewicht der erhaltene Verlauf $\Phi_{\Delta pMC}(m)$ mit Normalverläufen verglichen und zur Auswertung herangezogen wird.

17. Arbeitsverfahren nach den Ansprüchen 15 oder 16,
dadurch gekennzeichnet,
daß zusätzlich eine Auswertung der Kennfunktionen arteriolovenoläre Sauerstoff-Differenz $d = f(n)$, periphere Volumen-Koeffizienten $pVC_2 = f(n)$ und $pVC_3 = f(n)$, Veränderung der arteriellen Sauerstoff-Sättigung $\Delta O_2 = f(n)$, $T_H(n) \cdot FV(n) = f(n)$, $T_H(n)/FV(n) = f(n)$ erfolgt.

18. Arbeitsverfahren nach den Ansprüchen 1, 2 oder 3,
dadurch gekennzeichnet,
daß durch Bildung des Verhältnisses $T_H(n) / FV2_{NIR}(n)$ der aktuellen Messung zu demselben Verhältnis der Bezugsmessung die Veränderung des peripheren Strömungswiderstandes $\Delta PW$ bestimmbar ist, wobei gilt:

$$\Delta PW(n)\ [\%]\ =\ \frac{\dfrac{T_H(n)}{FV2_{NIR}(n)}\ \bigg/\ \begin{array}{l}\text{aktuelle}\\[2pt]\text{Messung}\end{array}}{\dfrac{T_H(n)}{FV2_{NIR}(n)}\ \bigg/\ \begin{array}{l}\text{Bezugs-}\\[2pt]\text{messung}\end{array}}\ \cdot\ 100$$

19. Arbeitsverfahren nach Anspruch 3,
dadurch gekennzeichnet,
daß die eingeführten Zustandsfunktionen $T_H = f(n)$, $FV = f(n)$, $pMC = f(n)$, $pVC = f(n)$, $\Delta O_2 = f(n)$, $d = f(n)$ nach dem Bildungsalgorithmus

$$\Phi_{xy}(m) = \overline{x(n)\ y(n\text{-}m)}$$

untereinander kreuzkorreliert werden, so daß aus den entstehenden Verläufen die Korrelationsfaktoren und auftretende Perioden $m_{Periode}$ ableitbar sind und diese mit Normalverläufen und -kennwerten verglichen werden.

20. Verfahren zum Feststellen der Auswirkungen von Pharmaka oder Drogen sowie zum Ableiten der Größen zur Patientenüberwachung unter Nutzung der Zustands- und Auswerteparameter, erhalten nach einem oder mehreren der Ansprüche 1 bis 19.

Fig. 1

Meßort
Fingerbeere
Sensorgrundkörper

δ S1 E S2

CMMD-System

S1...Sender1 (840nm)
S2...Sender2 (640nm)
E .... Empfänger
δ .... Temperatursensor

mikrovaskuläre
Blutvolumenpulsation

$x_{mikro \, ges}(t)$     $x_{II, III, ..., N}(t)$     $x_I(t)$

Pat.Nr.:22; ness0004.70s; 3.1.1995, 15:48Uhr

Kanal I

0 1 2 3 4 5 6 7 8 9 10 11 12 13 14 15 16 17 18 19 20 21 22 23 24 25 26 27 28 29 30 31 32

[s]

32 33 34 35 36 37 38 39 40 41 42 43 44 45 46 47 48 49 50 51 52 53 54 55 56 57 58 59 60 61 62 63 64

[s]

a)

**Fig. 2 a**

Zeitkurve

mittlere Volumenpulsfläche $\overline{F}$, wenn
N Volumenpulse während der Meßzeit von
64 s vorliegen:

$$\overline{F} = \frac{1}{N} \cdot \sum_{\nu=1}^{N} F_{\nu}$$

Kanal I

$x_I(t)$

$F_{20}$

$T_A$

$T_G$ $T_{AG}$

$T_D$

Dikrotie

$T_H$

21     22     23     24

16     17     18     19     20

[s]

**Beispiel:**
Fläche $F_{20}$ des Volumenpulses Nr 20 entspricht
einer Blutvolumenmenge dieses Pulses

b)

**Fig. 2b**

EP 0 913 120 A1

mikrovaskuläre Blutvolumenpulsation,
ermittelt mit der Wellenlänge 840 nm

Kanal 1

52      53      54      55      56

[s]

oxymetrisches Signal, ermittelt
mit der Wellenlänge 640 nm

Kanal 2

52      53      54      55      56

[s]

c)

Fig. 2c

Fig. 3

peripherer Mikrozirkulations-Koeffizient  pMC

**Fig. 4**

Fig. 5a

Fig. 5b

Pat.Nr.:43; mess0002.70s; 15.5.1996, 14:53Uhr │ T:34.0°C;

Mittelwert = 94,3%
Variabilität = 6,8%

Fig. 6

Fig. 7

Pat.Nr.:12; mess0009.70s; 3.4.1995, 14:04Uhr

**NIR-Signal**

Kanal1

0 1 2 3 4 5 6 7 8 9 10 11 12 13 14 15 16 17 18 19 20 21 22 23 24 25 26 27 28 29 30 31 32

[s]

pMC = 76,3% ± 19,7%

**Rot-Signal**

Kanal2

0 1 2 3 4 5 6 7 8 9 10 11 12 13 14 15 16 17 18 19 20 21 22 23 24 25 26 27 28 29 30 31 32

[s]

**Fig. 8**

Zeitkurve

Lunge,W.

Pat.Nr.:8; mess0005.70s; 9.11.1994, 17:08Uhr

**NIR-Signal**

Kanal 1

$T_{Atmung}$

0 1 2 3 4 5 6 7 8 9 10 11 12 13 14 15 16 17 18 19 20 21 22 23 24 25 26 27 28 29 30 31 32

[s]

**Rot-Signal**

Kanal 2

0 1 2 3 4 5 6 7 8 9 10 11 12 13 14 15 16 17 18 19 20 21 22 23 24 25 26 27 28 29 30 31 32

[s]

**Fig. 9**

**Fig. 10**

EP 0 913 120 A1

Herzperiode

Fig. 11

Korrelation Herzperiodendauer

Fig. 12

Histogramm Herzperiodendauer

Pat.Nr.:5; mess0019.70s; 2.3.1995, 11:43Uhr

Herzperiodendauer $T_H$ = 781 ms

Herzfrequenz = 77 [min$^{-1}$]

Streuung $s_{T_H\ mittel}$ = 20 ms

- Herzfrequenzvariabilität VHF = 2.6 %

Grenzen für Herzfrequenz
(Pulsfrequenz) nach
Sandoe und Sigurd

Tachykardie 0%    Border-line 1%    normaler Sinusrhythmus 99%    Bradykardie 0%

300  400  500  600  700  800  900  1000  1100  1200  1300  1400  1500  1600

Herzperiodendauer, Puls [ms]

150  120  100  86  75  67  60  55  50  46  43  40  38

Herzfrequenz, Puls [min$^{-1}$]

**Fig. 13**

Fig. 14

Fig. 15

Fig. 16

Korrelation Herzperiodendauer        6 KHK,NYHA 3

Pat.Nr.:6; mess0001.70s; 15.5.1996, 15:41Uhr | T:32.5°C;

**Fig. 17**

Relatives Füllungsvolumen 1.Art

Pat.Nr.:5; mess0019.70s; 2.3.1995, 11:43Uhr

relatives Füllungsvolumen FV

$$FV(n) = \overline{FV(n)} + \Delta FV(n)$$
$$= 1 + \Delta FV(n)$$

Pulsnummer $n$

-✗✗✗✗✗- Kanal1: MW = 1

-+++++- Kanal2: MW = 1

**Fig. 18**

**Relatives Füllungsvolumen 1.Art**      **3 Diabetes, Typ I WHO**

Pat.Nr.:3; mess0001.70s; 6.10.1993, 15:56Uhr

relatives Füllungsvolumen $FV$

langwellige Grundperioden

$\Delta FV$

angenäherter Verlauf des mittleren Füllungsvolumens am Meßort Fingerbeere

0 3 6 9 12 15 18 21 24 27 30 33 36 39 42 45 48 51 54 57 60 63 66 69 72 75 78 81 84 87 90

Pulsnummer n

Kanal1: MW = 1

**Fig. 19**

EP 0 913 120 A1

**Pat.Nr.:22; ness0004.70s; 3.1.1995, 15:48Uhr**

relatives Füllungsvolumen 1    **Bezugsmessung**
[ Volumen ]                     (Ruheventilation)

Maß für die Variabilität
des Blutfüllungsvolumens        Kanal 1

Atmungsperioden

Pulsnummer n

Kanal 1: Mittelwert = 1 Volumen
      Volumenvariabilität = 6,9%
**a)**    [ Normalbereich = 8%... 13%... 18% ]

**Pat.Nr.:22; ness0009.70s; 3.1.1995, 16:22Uhr | T:27.5°C;**

relatives Füllungsvolumen 2    Bezugsmessung 0004.70s

Bei Hyperventilation:
° Mittelwert Kanal 2 (oxym.Signal)= 0,45
  kleiner als Mittelwert Kanal 1 (Blutvol.)
  = 0,85, d.h. arterielle $O_2$-Sättigung im
  Meßgebiet ist angestiegen.
° Blutvolumen (Kanal 1) ist um 15%
  auf 0,85 gegenüber Ruheventilation
  reduziert.

Kanal 1

Kanal2

Pulsnummer n

Kanal 1: Mittelwert = 0,85 Volumen
**b)** Kanal 2: Mittelwert = 0,45 Volumen

# Fig. 20

Relatives Füllungsvolumen 1. Art

**Fig. 21**

Fig. 22

Fig. 23 a

Fig. 23b

Relatives Füllungsvolumen 2. Art                                    Pat3.stat

| Pat.Nr.:1; mess0031.70s; 25.9.1996, 10:32Uhr | T:25.5°C; |

Bezugsmessfile: MESS0016

relatives Füllungsvolumen 2
[Volumen]

$\Delta FV2 = FV2_{640nm} - FV2_{840nm}$

Gefäßdilatation
= 58 %

Mittelwerte $MW_{Kanal 1} = MW_{Kanal 2} = 1$
( Bezugsmessung, vor Therapie )

0  3  6  9  12 15 18 21 24 27 30 33 36 39 42 45 48 51 54 57 60 63 66 69 72 75 78

c)    Kanal 1: ××××× Mittelwert = 1.58 Volumen
      Kanal 2: +++++ Mittelwert = 1.79 Volumen          Pulsnummer n

Fig. 23c

Fig. 24

Beginn
O$_2$-Maskenatmung

Europäisches
Patentamt

**EUROPÄISCHER RECHERCHENBERICHT**

Nummer der Anmeldung

EP 97 11 8971

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y<br>A<br>A | WO 97 24980 A (CIRCUITRY SYSTEMS LTD.)<br>* Seite 3, Zeile 26 – Seite 5, Zeile 23 *<br>* Seite 10, Zeile 28 – Seite 11, Zeile 6 *<br>* Seite 11, Zeile 28 – Zeile 32 *<br>* Seite 12, Zeile 20 – Zeile 29 *<br>--- | 1,2<br>6,8,13<br>16,20 | A61B5/00 |
| Y | US 5 595 176 A (M. YAMAMAURA)<br>* Spalte 2, Zeile 49 – Spalte 3, Zeile 48 *<br>* Spalte 7, Zeile 3 – Spalte 8, Zeile 64 *<br>--- | 1,2 | |
| A<br>A | WO 93 16629 A (T. CADELL)<br>* Seite 7, Zeile 5 – Seite 8, Zeile 18 *<br>* Seite 14, Zeile 29 – Seite 16, Zeile 24 *<br>--- | 1,2,6<br>13,16,19 | |
| A | WO 91 11136 A (BOSTON ADVANCED TECHNOLOGIES, INC. )<br>* Seite 4, Zeile 1 – Seite 6, Zeile 28 *<br>* Seite 11, Zeile 1 – Zeile 25 *<br>----- | 1,2,20 | |

RECHERCHIERTE
SACHGEBIETE (Int.Cl.6)

A61B

Der vorliegende Recherchenbericht wurde für alle Patentansprüche erstellt

| Recherchenort | Abschlußdatum der Recherche | Prüfer |
|---|---|---|
| DEN HAAG | 30.März 1998 | Rieb, K.D. |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsatze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
   Dokument

EPO FORM 1503 03 82 (P04C03)